# EUROPEAN PATENT APPLICATION

(11) **EP 3 919 469 A1**
(43) Date of publication of application: **08.12.2021**
(21) Application number: 20747658.1
(22) Date of filing: 17.01.2020
(51) Int. Cl.: C07C 45/38, C07C 47/00, C07C 49/00, C07D 213/48, C07D 333/22

(54) **COPPER-CATALYZED METHOD FOR PREPARING ALDEHYDE OR KETONE COMPOUND BY OXIDIZING ALCOHOL BY USING OXYGEN AS OXIDANT, AND APPLICATION THEREOF**

(30) Priority: 29.01.2019 CN 201910084267
(71) Applicant: Fudan University, Shanghai 200433 (CN)
(72) Inventor: MA, Shengming, Shanghai 200433 (CN); ZHAI, Di, Shanghai 200433 (CN)
(74) Representative: Barker Brettell LLP
(86) International application number: PCT/CN2020/072732
(87) International publication number: WO 2020/156238

(57) **Abstract**

The present invention discloses a method for preparing aldehydes or ketones via aerobic oxidation of alcohols with the copper salts and nitroxide radicals as catalysts. Both oxygen and air could be used as oxidants, after 4 to 48 hours of reaction in an organic solvent at room temperature, the alcohols are efficiently oxidized to the corresponding aldehydes or ketones. The present invention has the following advantages: easy to operate, refraining from using chlorides which are corrosive to equipment, readily available raw materials and reagents, mils reaction conditions, the broad substrate scope, good functional group tolerance, convenient purification, environmentally friendly and no pollution. Thus, the method is suitable for industrial production.

## Description

### TECHNICAL FIELD

The present invention belongs to the technical field of chemical synthesis, particularly to a copper-catalyzed method and application for preparing aldehydes or ketones by oxidizing alcohols with oxygen as an oxidant.

### BACKGROUND OF THE INVENTION

As a kind of important raw material for organic synthesis, aldehydes and ketones are widely used in the industry and scientific research area. Oxidation is one of the most basic and important chemical reactions. In industry, aldehydes and ketones are often prepared by the oxidation of alcohols. Therefore, the development of mild conditions, environment-friendly, efficient and convenient catalytic oxidation system has a broad application prospect. Traditional oxidation methods of alcohol compounds often need to use stoichimetric oxidants and produce equivalent industrial waste, which brings great harm to the ecological environment and is not suitable for large-scale industrial production ( Chromium Oxidations in Organic Chemistry; Springer: Berlin, 1984; Regen, S. L.; Koteel, C. J. Am. Chem. Soc. 1977, 99, 3837; Griffith, W. P. Chem. Soc. Rev. 1992, 21, 179; Caron, S.; Dugger, R. W.; Ruggeri, S. G.; Ragan, J. A.; Ripin, D. H. B. Chem. Rev. 2006, 106, 2943.) . Therefore, a series of metal-catalyzed oxidation methods using oxygen or oxygen in the air as oxidants have been developed (Marko, I. E.; Giles, P. R.; Tsukazaki, M.; Brown, S. M.; Urch, C. J. Science 1996, 274, 2044; Dijksman, A.; Arends, I.W. C. E.; Sheldon, R. A. Chem. Commun. 1999, 1591; Shibuya, M.; Tomizawa, M.; Suzuki, I.; Iwabuchi, Y J. Am. Chem. Soc. 2006, 128, 8412; Piera, J.; Backvall, J. E. Angew. Chem. Int. Ed. 2008, 47, 3506; Steves, J. E.; Stahl, S. S. J. Am. Chem. Soc. 2013, 135, 15742; Guan, M.; Wang, C.; Zhang, J.; Zhao, Y RSC Adv. 2014, 4, 48777; Jiang, J.-A.; Du, J.-L.; Wang, Z.-G.; Zhang, Z.-N.; Xu, X.; Zheng, G.-L.; Ji, Y.-F. Tetrahedron Lett. 2014, 55, 1677; Zhang, G.; Yang, C.; Liu, E.; Li, L.; Golen, J. A.; Rheingold, A. L. RSC Adv. 2014, 4, 61907; Wang, D.; Weinstein, A. B.; White, P. B.; Stahl, S. S. Chem. Rev. 2018, 118, 2636). TEMPO, as a cheap, stable and efficient oxygen radical, plays an important role in the oxidation system with iron or copper salts as catalysts (Gamez, P.; Arends, I. W.C.E.; Reedijk, J.; Sheldon, R. A. Chem. Commun. 2003, 2414; Ma, S.; Liu, J.; Li, S.; Chen, B.; Cheng, J.; Kuang, J.; Liu, Y; Wan, B.; Wang, Y; Ye, J.; Yu, Q.; Yuan, W.; Yu, S. Adv. Synth. Catal. 2011**,** Hoover, J. M.; Stahl, S. S. J. Am. Chem. Soc. 2011, 133, 16901; 353, 1005; Liu, J.; Ma, S. Org. Lett. 2013, 15, 5150; Liu, J.; Xie, X.; Ma, S. Synthesis 2012, 44, 1569; Jiang, X.; Zhang, J.; Ma, S. J. Am. Chem. Soc. 2016, 138, 8344; Jiang, X.; Ma, S. Synthesis 2018, 50, 1629) .

### SUMMARY OF THE INVENTION

The present invention overcomes the disadvantages of using heavy metals as catalysts or chlorides, harsh reaction conditions, long reaction time, limited types of catalytic substrates, use of high temperature and high pressure, etc. in the existing metal-catalyzed oxygen oxidation technology, and provides a method for preparing aldehydes or ketones by oxidizing alcohols with oxygen under 1 atmospheric pressure, using copper salt and nitroxide radicals as catalysts, oxygen is used as oxidant. The method of the present invention has wide sources of raw materials, can avoid the discharge of a large number of chemical reaction wastes, can greatly reduce the cost of the oxidation reaction, and has the beneficial effects of mild reaction, high reaction efficiency, suitability for large-scale production, low cost, and ecological and environmental protection.

The purpose of the present invention is to provide a method with oxygen as an oxidant with mild reaction conditions, high efficiency, low cost and green for preparing aldehydes or ketones by catalytic oxidation of alcohol.

The object of the present invention is achieved by using the following solution:
A method for preparing aldehydes or ketones compounds by oxidizing alcohols with oxygen as an oxidant, including at 0-100°C, in an organic solvent, using the alcohol shown in formula (a) as raw material, oxygen or oxygen in the air is used as oxidant, copper salts and nitroxide radicals are used as catalysts, reacting 4-48 hours to oxidize the alcohol to produce aldehyde or ketone compounds shown in formula (b), the reaction process has the following reaction equation (1): 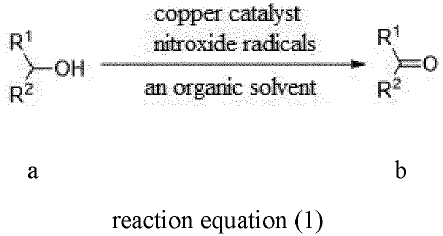 wherein, R¹ and R² is a hydrogen, an alkyl, an alkyl with functional groups, a cycloalkyl, a phenyl, an aryl, a heterocyclic group, an ethynyl, an alkynyl with functional groups, a vinyl, an alkenyl with functional groups, an allenyl, an allenyl with functional groups; the said aryl is phenyl, naphthyl, thiophene, furan, pyrrole with electron-donating or electron-withdrawing substituents at the ortho, meta, and para positions; the said heterocyclic group is thienyl, furyl or pyridyl, or thiophene, furan or pyridine with electron-donating or electron-withdrawing substituents.

Preferably, R¹ and R² is a C1-C20 alkyl, a C1-C20 alkyl with functional groups, a C3-C8 cycloalkyl, a phenyl, an aryl, a heterocyclic group, an ethynyl, an alkynyl with functional groups, a vinyl, an alkenyl with functional groups, an allenyl, an allenyl with functional groups; the said aryl is phenyl, thiophene, furan, pyrrole with substituents at the ortho, meta, and para positions; the said substituent is selected from C1-C5 alkyl, ester group (the said ester group including methoxycarbonyl, ethoxycarbonyl), hydroxyl group, acyl group (the said acyl group including formyl, acetyl, benzoyl), acyloxy group (the said acyloxy group including formyloxy, acetoxy, benzoyloxy), nitro group, halogen, carboxyl group, cyano group, methoxyl group; the said heterocyclic group is thienyl, furyl or pyridyl, or thiophene, furan or pyridine with electron-donating or electron-withdrawing substituents;
wherein, the C1-C20 alkyl with functional group, said the functional group is selected from carbon-carbon double bond, carbon-carbon triple bond, ester group (the said ester group including methoxycarbonyl, ethoxycarbonyl), acyl group (the said acyl group including formyl, acetyl, benzoyl), acyloxy group (the said acyloxy group including formyloxy, acetoxy, benzoyloxy), amido (the said amido including acetamide, benzamide), halogen, carboxyl group, cyano group, phenyl, aryl, thienyl, furyl; the alkynyl with functional group, the alkenyl with functional group, and the allenyl with functional group, said the functional group is selected from C1-C20 alkyl, C3-C6 cycloalkyl, carbon-carbon double bond, carbon-carbon triple bond, ester group (the said ester group including methoxycarbonyl, ethoxycarbonyl), acyl group (the said acyl group including formyl, acetyl, benzoyl), acyloxy group (the said acyloxy group including formyloxy, acetoxy, benzoyloxy), amido (the said amido including acetamide, benzamide), halogen, carboxyl group, cyano group, phenyl, aryl, thienyl, furyl, silicon group; all aryl groups in the above definition of functional group is a phenyl, thiophene, furan, pyrrole with substituents at the ortho, meta, and para positions, the said substituent is selected from C1-C5 alkyl, ester group (the said ester group including methoxycarbonyl, ethoxycarbonyl), hydroxyl group, acyl group (the said acyl group including formyl, acetyl, benzoyl), acyloxy group (the said acyloxy group including formyloxy, acetoxy, benzoyloxy), nitro group, halogen, carboxyl group, cyano group, methoxyl group;
more preferably, R¹ and R² is a C1-C16 alkyl, a C1-C16 alkyl with functional groups, a C3-C6 cycloalkyl, a phenyl, an aryl (the said aryl is a phenyl with substituents at the ortho, meta, and para positions, the said substituent is selected from methoxycarbonyl, hydroxyl group, nitro group, chlorine, bromine, iodine, cyano, methyl, methoxy), a heterocyclic group (the said heterocyclic group is thienyl, furyl), an ethynyl, an alkynyl with functional groups, a vinyl, an alkenyl with functional groups, an allenyl, an allenyl with functional groups;
wherein, the C1-C16 alkyl with functional groups, the said functional group is selected from carbon-carbon double bond, carbon-carbon triple bond, methoxycarbonyl, ethoxycarbonyl, acetyl, benzoyl, acetoxy, benzoyloxy, benzamide, halogen, carboxyl group, cyano group, phenyl, aryl (the said aryl is a phenyl with substituents at the ortho, meta, and para positions, the said substituent is selected from methoxycarbonyl, hydroxyl group, nitro group, chlorine, bromine, iodine, cyano, methyl, methoxy), thienyl, furyl; the alkynyl with functional groups, the alkenyl with functional groups, and the allenyl with functional groups, said the functional group is selected from C1-C16 alkyl, C3-C6 cycloalkyl, phenyl, p-bromophenyl, o-hydroxyphenyl, thienyl, furyl, trimethylsilyl.

As a further improvement, the specific operation steps of the present invention are as follows:
1) inserting an oxygen balloon into the dry reaction tube, pumping air three times, and adding a copper catalyst, a nitroxide radical, an alcohol organic solvent solution in sequence, or using air to supplement oxygen, or airflow, putting the reaction tube in the 25°C oil bath and stirring for 4-48 hours;
2) after the completion of the reaction in step (1), raising the reaction tube from the oil bath, filtering the mixture with silica gel short column, washing with a certain amount of diethyl ether, concentrating, and subjecting to the flash column chromatography, so as to obtain the aldehyde or ketone compounds; said the diethyl ether is based on the amount of alcohol shown in formula (a), and the dosage of the diethyl ether is 3.75-75 mL/mmol; preferably, is 75 mL/mmol.

As a further improvement, the organic solvent used in the present invention is any one or more of benzene, toluene, dichloromethane (DCM), 1,2-dichloroethane (DCE), 1,1-dichloroethane, 1,2-dichloropropane, 1,3-dichloropropane, nitromethane (MeNO₂), diethyl ether (Et₂O), ethylene glycol dimethyl ether, tetrahydrofuran (THF) or acetonitrile (MeCN); preferably, is acetonitrile or 1,2-dichloroethane; wherein, said the organic solvent is based on the amount of alcohol shown in formula (a), and the dosage of the organic solvent is 1.0-10.0 mL/mmol; preferably, is 4.0 mL/mmol.

As a further improvement, the copper salts used in the present invention are any one or more of tetrakiscopper hexa-fluorophosph (Cu(MeCN)₄PF₆), cuprous chloride, copper bromide, cuprous iodide, copper acetate or copper nitrate trihydrate; preferably, is copper nitrate trihydrate (Cu(NO₃)₂•3H₂O); said the copper salt is based on the amount of alcohol shown in formula (a), and the dosage of the copper salt is 0.025-0.1 mmol/mmol; preferably, is 0.1 mmol/mmol.

As a further improvement, the nitroxide radicals used in the present invention are any one or more of 2,2,6,6-tetramethylpiperidine oxide, 4-hydroxy-2,2,6,6-tetramethylpiperidine oxide, 4-methoxy-2,2,6,6-tetramethylpiperidine oxide, 4-acetylamino -2,2,6,6-tetramethylpiperidine oxide, 4-oxy-2,2,6,6-tetramethylpiperidine oxide, 4-amino-2,2,6,6-tetramethylpiperidine oxide, N-hydroxymaleimide, 9-azabicyclo [3.3.1] nonane nitroxide radical, 2-azaadamantane nitroxide radical; preferably, is 2,2,6,6-tetramethylpiperidine oxide (TEMPO); said the nitroxide radical is based on the amount of alcohol shown in formula (a), and the dosage of the nitroxide radical is 0.025-0.1 mmol/mmol; preferably, is 0.1 mmol/mmol.

The present invention proposes a method at room temperature, in an organic solvent, copper nitrate trihydrate (Cu(NO₃)₂•3H₂O), 2,2,6,6-tetramethylpiperidine oxide (TEMPO) or 4-hydroxy-2,2,6,6-tetramethylpiperidine oxide (4-OH-TEMPO) are used as catalysts, oxygen or oxygen in the air are used as oxidant, for preparing aldehydes or ketones by oxidizing alcohols. Through the method of the present invention, in the atmosphere of oxygen in the air or pure oxygen under normal pressure, alcohols with functional groups such as carbon-carbon single bond, carbon-carbon double bonds, carbon-carbon triple bonds and so on can be selectively oxidized, corresponding aldehydes and ketones can be prepared from primary alcohols or secondary alcohols. The present invention has the advantages of simple operations, mild reaction conditions, high yield, wide substrate universality, convenient separation and purification, environmental friendliness and no pollution, and is suitable for industrial production.

Taking the catalyst 2,2,6,6-tetramethylpiperidine oxide (TEMPO) as an example, the present invention proposes the following possible mechanism for the reaction:
TEMPO first interacts with Cu²⁺ to form intermediate 1, which interacts with alcohol substrate to form intermediate 2, and intermediate 2 simultaneously undergoes β-H elimination and reduction elimination, and then generates the corresponding aldehyde or ketone compounds, TEMPOH and Cu⁺ at the same time, and then Cu⁺ is oxidized by NO₂ to produce Cu²⁺, TEMPOH is oxidized by Cu²⁺ to produce TEMPO and Cu⁺, realizing the catalytic cycle of TEMPO, and the produced Cu⁺ is reoxidized by NO₂ to Cu²⁺, thus realizing the catalytic cycle of copper, the catalytic cycle of Cu⁺ and Cu²⁺ is realized synchronously with the cycle of NO₂ and NO, while the cycle of NO₂ and NO is realized by the oxidation of NO by the addition of oxygen. The specific mechanism is shown in the following formula.

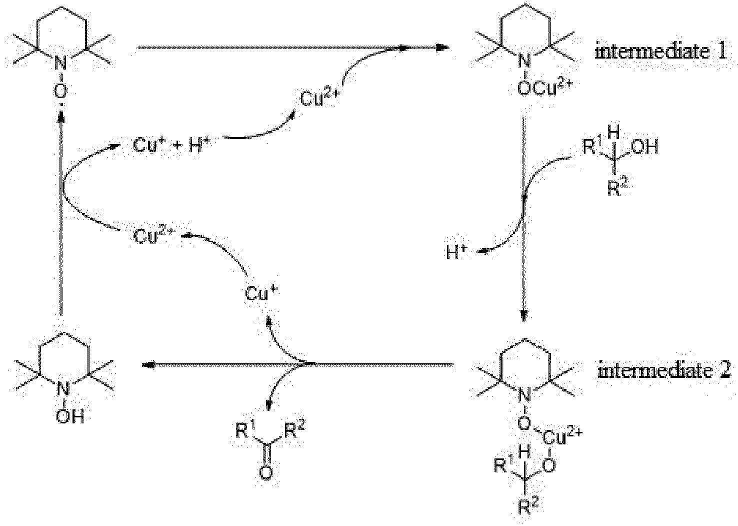

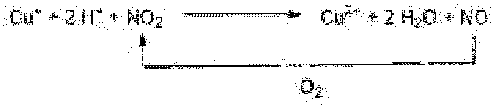

The present invention has the advantage of wide substrate universality. The copper salts, TEMPO are used as catalysts, which can not only catalyze and oxidize the common alcohol, benzyl alcohol, allyl alcohol and so on, but also can be used to catalyze and oxidize a series of alcohols with a more complex structure such as propargyl alcohol and allenol. The present invention has high catalytic efficiency, for example, when the amount of the catalyst is as low as 2.5 mol% (based on the amount of the alcohol shown in formula (a)), the corresponding aldehyde or ketone compounds can be generated. The present invention overcomes the disadvantages of the prior art requiring the use of relatively expensive heavy metal salts as catalysts, harsh reaction conditions, long reaction time, and limited types of catalytic substrates. The method proposed by the present invention can be used for laboratory synthesis as well as large-scale industrial production.

Compared with the traditional oxidation method of alcohol compounds, the by-product of the present invention is water, no equivalent or excessive oxidizing agent is required, and no equivalent or excessive waste liquid and waste residue are generated. Compared with the existing oxidation reactions in which other metal salts and nitroxide radicals are used as catalysts, the present invention has cheap and easily available raw materials and mild reaction conditions. Different from the existing oxidation method, the present invention creatively avoids the addition of ligands and metal chlorides, does not generate hydrogen chloride or other waste liquids that have corrosive effects on industrial reactors, and can greatly reduce industrial production costs.

The present invention uses low-cost, widely available oxygen or air as the oxidant instead of the chemical oxidant used in the traditional oxidant system. Therefore, the reaction conditions for the catalytic oxidation of alcohol to the corresponding aldehyde or ketone compounds in the present invention are extremely mild, and can be carried out only under room temperature, normal pressure, and neutral conditions, and the operation is simple, convenient and easy to control. The reaction can proceed smoothly under the conditions of room temperature and an oxygen pressure of 1 atmospheric pressure. Since the oxidant used in the reaction process is oxygen or oxygen in the air, and the by-product is water, the entire reaction process hardly generates any waste gas, waste liquid, and slag that are harmful to the environment. It is a green chemical synthesis method. The post-treatment process of the present invention is simple, the product yield is high (the highest can be completely converted), and the production cost is effectively reduced.

### PREFERRED EMBODIMENTS OF THE INVENTION

With reference to the following specific embodiments, the present invention will be further described in detail, and the protection of the present invention is not limited to the following embodiments. Without departing from the spirit and scope of the inventive concept, changes and advantages that those skilled in the field can think of are all included in the present invention, and the appended claims are the protection scope. The processes, conditions, reagents, experimental methods and so on, for implementing the present invention, except for the content specifically mentioned below, are common knowledge and common knowledge in the field, and the present invention has no special limitations. The following examples are helpful to understand the present invention, but do not limit the protection scope of the present invention.
wherein, in the following example reaction formula, "equiv" represents equivalent; "mol" represents mole; "Cu(NO₃)₂•3H₂O" represents copper nitrate trihydrate(II); "TEMPO" represents 2,2,6,6-tetramethylpiperidine oxide; "4-OH-TEMPO" represents 4-hydroxy-2,2,6,6-tetramethylpiperidine oxide; "MeCN" represents acetonitrile; "DCE" represents 1,2-dichloroethane; "NMP" represents N-methylpyrrolidone; "MeNO₂" represents nitromethane; "THF" represents tetrahydrofuran; "Et₂O" represents diethyl ether; "DCM" represents dichloromethane; "Cu(MeCN)₄PF₆" represents tetrakiscopper hexa-fluorophosph; "CuCl" represents cuprous chloride(I); "CuBr₂" represents copper bromide(II); "CuI" represents cuprous iodide(I); "Cu(OAc)₂" represents copper acetate (II); "rt" represents the room temperature; "O₂ balloon" represents the reaction is carried out in the oxygen atmosphere provided by the oxygen balloon; "air bag" represents the reaction is carried out in the air atmosphere provided by the air bag; "O₂ bag" represents the reaction is carried out in the atmosphere of oxygen supplement in the oxygen bag; "min" represents minute; "h" represents hour; the boiling range of petroleum ether is 60-90°C; the NMR yield is determined by ¹H NMR; the internal standard is dibromomethane, and the mesh number of silica gel is 300-400.

### Example 1

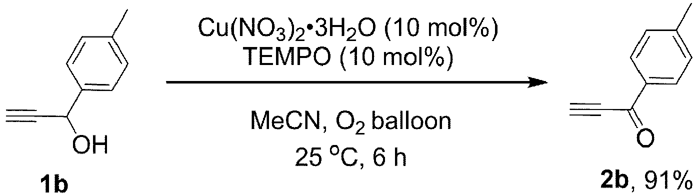

An oxygen balloon was inserted into the dry reaction tube, pumped O₂ for three times, Cu(NO₃)₂•3H₂O (24.6 mg, 0.1 mmol), TEMPO (16.2 mg, 0.1 mmol), and 1b (146.4 mg, 1.0 mmol) of MeCN solution (4 mL) were added sequentially. The reaction tube was stirred at 25 °C for 6 h. The mixture solution was filtered through a short column of silica gel (2cm), and washed with ethyl ether, rotary evaporation to remove the solvent. The mixture solution was separated and purified by column chromatography on silica gel (eluent: petroleum ether/ethyl ether = 60/1), to afford **2b** (130.7 mg, 91%): white solid. Melting point: 44.2-44.7 °C (petroleum ether/ethyl acetate recrystallization); **¹H NMR** (400 MHz, CDCl₃): δ = 8.06 (d, *J =* 8.4 Hz, 2 H, ArH), 7.29 (d, *J* = 8.0 Hz, 2 H, ArH), 3.40 (s, 3 H, CH₃), 2.44 (s, 1 H, ≡CH); ¹³C **NMR** (100 MHz, CDCl₃): δ = 177.0, 145.7, 133.8, 129.8, 129.4, 80.34, 80.28, 21.8; **MS** (70 eV, EI) *m*/*z* (%): 144 (M⁺, 74.75), 115 (100); **IR** (neat): v = 3257, 2090, 1632, 1594, 1459, 1404, 1310, 1246, 1167, 1117 cm⁻¹.

### Example 2

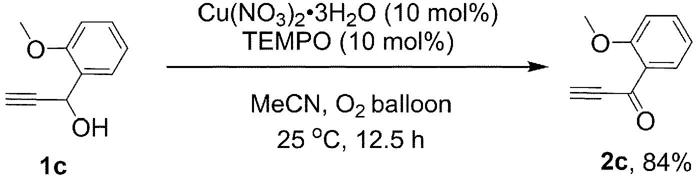

Operations were conducted by referring to Example 1. Cu(NO₃)₂•3H₂O (24.0 mg, 0.1 mmol), TEMPO (16.1 mg, 0.1 mmol), **1c** (161.8 mg, 1.0 mmol), MeCN (4 mL), reacted 12.5 hours to afford **2c** (134.0 mg, 84%) (eluent: petroleum ether/ethyl acetate = 30/1): oily liquid; **¹H NMR** (400 MHz, CDCl₃): δ = 8.06 (dd, *J*₁ = 7.8 Hz, *J*₂ = 1.8 Hz, 1 H, ArH), 7.55 (td, *J*₁ = 8.0 Hz, *J*₂ = 1.8 Hz, 1 H, ArH), 7.07-6.97 (m, 2 H, ArH), 3.94 (s, 3 H, CH₃), 3.37 (s, 1 H, ≡CH); **¹³C NMR** (100 MHz, CDCl₃): δ = 175.8, 159.8, 135.4, 132.9, 125.5, 120.1, 112.0, 81.9, 79.5, 55.6; **MS** (70 eV, EI) *m*/*z* (%): 161 (M⁺+1, 7.26), 160 (M⁺, 64.37), 131 (100); **IR** (neat): v = 3234, 2089, 1647, 1595, 1573, 1484, 1462, 1434, 1286, 1253, 1224, 1164, 1116, 1019 cm⁻¹.

### Example 3

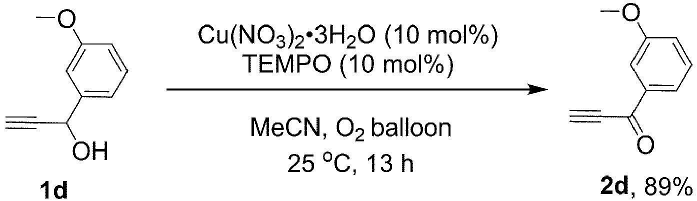

Operations were conducted by referring to Example 1. Cu(NO₃)₂•3H₂O (24.1 mg, 0.1 mmol), TEMPO (16.3 mg, 0.1 mmol), **1d** (162.2 mg, 1.0 mmol), MeCN (4 mL), reacted 13 hours to afford **2d** (142.6 mg, 89%) (eluent: petroleum ether/ethyl acetate = 30/1): oily liquid; **¹H NMR** (400 MHz, CDCl₃): δ = 7.80 (d, *J =* 7.6 Hz, 1 H, ArH), 7.64 (s, 1 H, ArH), 7.42 (t, *J =* 8.0 Hz, 1 H, ArH), 7.19 (dd, *J*₁ = 8.4 Hz, *J*₂ *=* 2.0 Hz, 1 H, ArH), 3.87 (s, 3 H, CH₃), 3.43 (s, 1 H, ≡CH); **¹³C NMR** (100 MHz, CDCl₃): δ = 177.1, 159.8, 137.4, 129.7, 122.9, 121.4, 112.8, 80.6, 80.3, 55.4; **MS** (70 eV, EI) *m*/*z* (%): 161 (M⁺+1, 11.54), 160 (M⁺, 100); **IR** (neat): v = 3250, 2094, 1644, 1595, 1581, 1485, 1429, 1323, 1264, 1207, 1177, 1021, 1012 cm⁻¹.

### Example 4

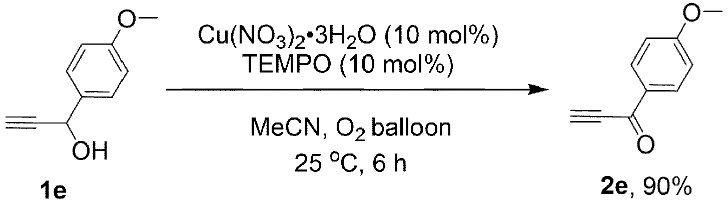

Operations were conducted by referring to Example 1. Cu(NO₃)₂•3H₂O (24.6 mg, 0.1 mmol), TEMPO (16.0 mg, 0.1 mmol), **1e** (161.8 mg, 1.0 mmol), MeCN (4 mL), reacted 6 hours to afford **2e** (143.6 mg, 90%) (eluent: petroleum ether/ethyl acetate = 25/1): white solid. Melting point: 85.5-86.5 °C (petroleum ether/ethyl acetate recrystallization); **¹H NMR** (400 MHz, CDCl₃): δ = 8.13 (d, *J =* 9.2 Hz, 2 H, ArH), 6.97 (d, *J =* 8.8 Hz, 2 H, ArH), 3.90 (s, 3 H, CH₃), 3.38 (s, 1 H, ≡CH); **¹³C NMR** (100 MHz, CDCl₃): δ = 175.8, 164.7, 132.0, 129.4, 113.8, 80.3, 80.1, 55.5; **MS** (70 eV, EI) *m*/*z* (%):161 (M⁺+1, 11.17), 160 (M⁺, 100); **IR** (neat): v = 3248, 2091, 1638, 1596, 1570, 1507, 1421, 1254, 1168, 1116, 1022, 1008 cm⁻¹.

### Example 5

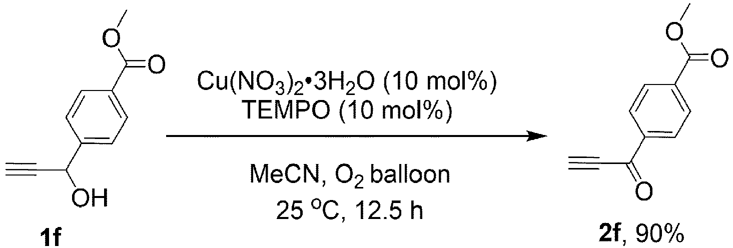

Operations were conducted by referring to Example 1. Cu(NO₃)₂•3H₂O (24.0 mg, 0.1 mmol), TEMPO (15.8 mg, 0.1 mmol), **If** (190.2 mg, 1.0 mmol), MeCN (4 mL), reacted 12.5 hours to afford **2f** (170.3 mg, 90%) (eluent: petroleum ether/ethyl acetate = 30/1): white solid. Melting point: 117.0-118.3 °C (petroleum ether/ethyl acetate recrystallization); **¹H NMR** (400 MHz, CDCl₃): δ = 8.22 (d, *J =* 8.4 Hz, 2 H, ArH), 8.16 (d, *J =* 8.4 Hz, 2 H, ArH), 3.97 (s, 3 H, CH₃), 3.53 (s, 1 H, ≡CH); **¹³C NMR** (100 MHz, CDCl₃): δ = 176.6, 165.9, 139.0, 135.0, 129.8, 129.4, 81.8, 80.0, 52.5; **MS** (70 eV, EI) *m*/*z* (%):189 (M⁺+1, 5.41), 188 (M⁺, 41.74), 157 (100); **IR** (neat): v = 3217, 2096, 1717, 1636, 1606, 1437, 1275, 1233, 1195, 1119, 1006 cm⁻¹.

### Example 6

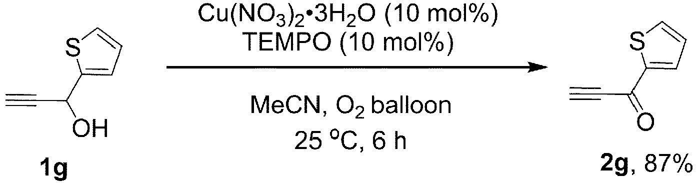

Operations were conducted by referring to Example 1. Cu(NO₃)₂•3H₂O (24.4 mg, 0.1 mmol), TEMPO (15.8 mg, 0.1 mmol), **1g** (137.8 mg, 1.0 mmol), MeCN (4 mL), reacted 6 hours to afford **2g** (118.4 mg, 87%) (eluent: petroleum ether/ethyl acetate = 30/1): white solid. Melting point: 32.4-34.0 °C (petroleum ether/ethyl acetate recrystallization); **¹H NMR** (400 MHz, CDCl₃): δ = 7.97 (dd, *J*₁ = 3.8 Hz, *J*₂ = 0.6 Hz, 1 H, ArH), 7.75 (dd, *J*₁ = 4.8 Hz, *J*₂ = 0.8 Hz, 1 H, ArH), 7.18 (t, *J* = 4.2 Hz, 1 H, ArH), 3.37 (s, 1 H, ≡CH); **¹³C NMR** (100 MHz, CDCl₃): δ = 169.0, 144.0, 136.1, 135.9, 128.4, 79.8, 79.3; **MS** (70 eV, EI) *m*/*z* (%): 136 (M⁺, 100), 108 (95.16); **IR** (neat): v = 3244, 3103, 2095, 1616, 1512, 1406, 1354, 1266, 1231, 1201, 1082, 1051 cm⁻¹.

### Example 7

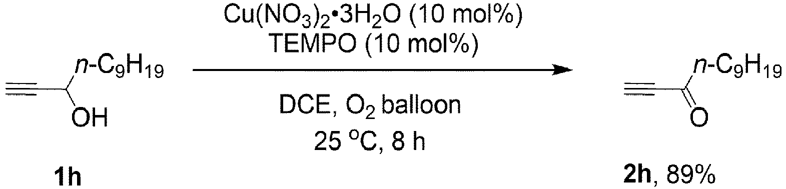

Operations were conducted by referring to Example 1. Cu(NO₃)₂•3H₂O (24.0 mg, 0.1 mmol), TEMPO (16.0 mg, 0.1 mmol), **1h** (182.1 mg, 1.0 mmol), DCE (4 mL), reacted 8 hours to afford **2h** (160.4 mg, 89%) (eluent: petroleum ether/ethyl ether = 40/1): oily liquid; **¹H NMR** (400 MHz, CDCl₃): δ = 3.20 (s, 1 H, CH), 2.58 (t, *J=* 7.4 Hz, 2 H, CH₂), 1.73-1.60 (m, 2 H, CH₂), 1.40-1.16 (m, 12 H, 6×CH₂), 0.88 (t, *J =* 6.8 Hz, 3 H, CH₃); **¹³C NMR** (100 MHz, CDCl₃): δ = 187.5, 81.4, 78.2, 45.4, 31.8, 29.3, 29.22, 29.17, 28.8, 23.7, 22.6, 14.0.

### Example 8

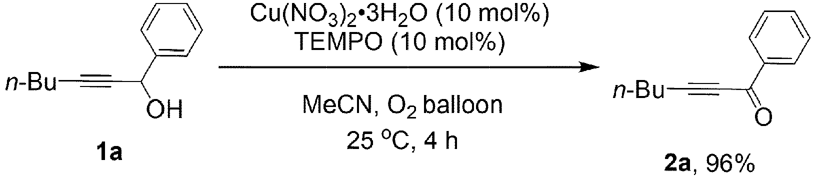

Operations were conducted by referring to Example 1. Cu(NO₃)₂•3H₂O (23.9 mg, 0.1 mmol), TEMPO (16.1 mg, 0.1 mmol), **1a** (187.2 mg, 1.0 mmol), MeCN (4 mL), reacted 4 hours to afford **2a** (177.4 mg, 96%) (eluent: petroleum ether/ethyl ether = 30/1): oily liquid; **¹H NMR** (400 MHz, CDCl₃): δ = 8.14 (d, *J =* 7.2 Hz, 2 H, ArH), 7.60 (t, *J =* 7.2 Hz, 1 H, ArH), 7.48 (t, *J =* 7.8 Hz, 2 H, ArH), 2.51 (t, *J =* 70 Hz, 2 H, CH₂), 1.74-1.58 (m, 2 H, CH₂), 1.58-1.42 (m, 2 H, CH₂), 0.97 (t, *J =* 7.4 Hz, 3 H, CH₃); **¹³C NMR** (100 MHz, CDCl₃): δ = 178.2, 136.9, 133.8, 129.5, 128.4, 96.8, 79.6, 29.8, 22.0, 18.8, 13.4; **MS** (70 eV, EI) *m*/*z* (%): 186 (M⁺, 12.46), 144 (100); **IR** (neat): v = 2957, 2933, 2868, 2232, 2200, 1641, 1588, 1452, 1314, 1257, 1172 cm⁻¹.

### Example 9

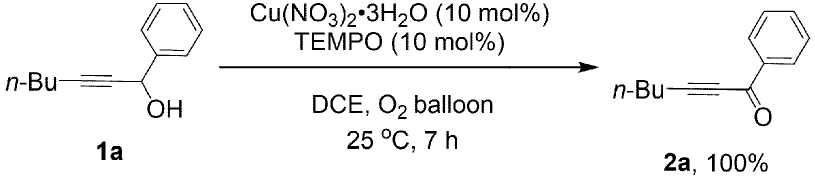

Operations were conducted by referring to Example 1. Cu(NO₃)₂•3H₂O (24.0 mg, 0.1 mmol), TEMPO (16.0 mg, 0.1 mmol), **1a** (188.0 mg, 1.0 mmol), DCE (4 mL), reacted 7 hours to afford 2a (185.2 mg, 100%) (eluent: petroleum ether/ethyl ether = 40/1): oily liquid; **¹H NMR** (400 MHz, CDCl₃): δ = 8.18-8.10 (m, 2 H, ArH), 7.63-7.57 (m, 1 H, ArH), 7.48 (t, *J=* 7.8 Hz, 2 H, ArH), 2.51 (t, *J=* 7.0 Hz, 2 H, CH₂), 1.74-1.58 (m, 2 H, CH₂), 1.58-1.42 (m, 2 H, CH₂), 0.97 (t, *J =* 7.6 Hz, 3 H, CH₃); **¹³C NMR** (100 MHz, CDCl₃): δ = 178.1, 136.8, 133.8, 129.4, 128.4, 96.7, 79.6, 29.7, 22.0, 18.8, 13.4.

### Example 10

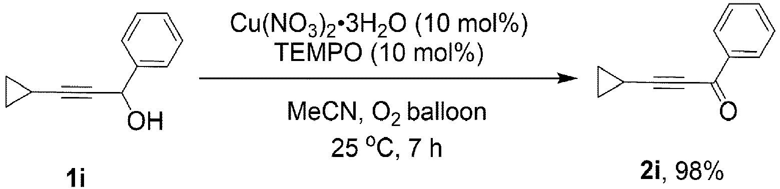

Operations were conducted by referring to Example 1. Cu(NO₃)₂•3H₂O (24.2 mg, 0.1 mmol), TEMPO (15.8 mg, 0.1 mmol), **1i** (172.2 mg, 1.0 mmol), MeCN (4 mL), reacted 7 hours to afford **2i** (166.6 mg, 98%) (eluent: petroleum ether/ethyl ether = 30/1): oily liquid; **¹H NMR** (400 MHz, CDCl₃): δ = 8.16-8.05 (m, 2 H, ArH), 7.59 (t, *J* = 7.4 Hz, 1 H, ArH), 7.47 (t, *J* = 7.6 Hz, 2 H, ArH), 1.64-1.48 (m, 1 H, CH), 1.12-0.96 (m, 4 H, CH₂CH₂); **¹³C NMR** (100 MHz, CDCl₃): δ = 177.7, 136.8, 133.6, 129.2, 128.3, 101.0, 75.4, 9.7, -0.2; **MS** (70 eV, EI) *m*/*z* (%):170 (M⁺+1, 10.75), 170 (M⁺, 70.44), 141 (100); **IR** (neat): v = 2207, 1635, 1596, 1579, 1449, 1356, 1311, 1264, 1172, 1046, 1023 cm⁻¹.

### Example 11

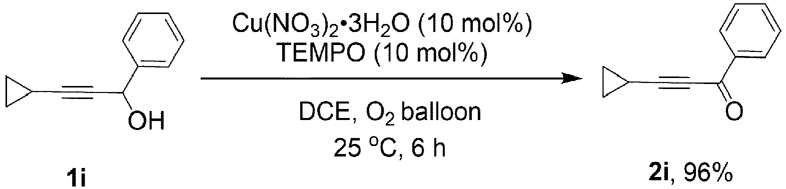

Operations were conducted by referring to Example 1. Cu(NO₃)₂•3H₂O (24.2 mg, 0.1 mmol), TEMPO (16.0 mg, 0.1 mmol), **1i** (172.3 mg, 1.0 mmol), DCE (4 mL), reacted 6 hours to afford **2i** (163.5 mg, 96%) (eluent: petroleum ether/ethyl ether = 30/1): oily liquid; **¹H NMR** (400 MHz, CDCl₃): δ = 8.20-8.05 (m, 2 H, ArH), 7.65-7.54 (m, 1 H, ArH), 7.47 (t, *J =* 7.6 Hz, 2 H, ArH), 1.66-1.48 (m, 1 H, CH), 1.13-0.94 (m, 4 H, CH₂CH₂); **¹³C NMR** (100 MHz, CDCl₃): δ = 177.8, 136.8, 133.6, 129.3, 128.3, 101.0, 75.4, 9.8, -0.1.

### Example 12

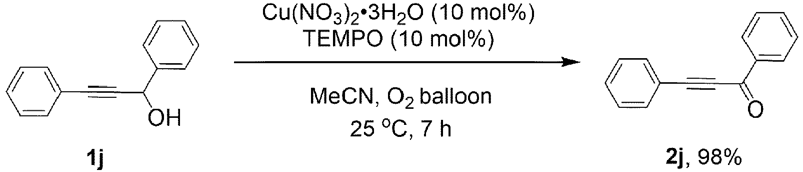

Operations were conducted by referring to Example 1. Cu(NO₃)₂•3H₂O (24.4 mg, 0.1 mmol), TEMPO (16.1 mg, 0.1 mmol), **1j** (209.8 mg, 1.0 mmol), MeCN (4 mL), reacted 7 hours to afford **2j** (203.4 mg, 98%) (eluent: petroleum ether/ethyl ether = 30/1): white solid. Melting point: 45.3-46.6 °C (petroleum ether/ethyl acetate recrystallization); **¹H NMR** (400 MHz, CDCl₃): δ = 8.23 (d, *J =* 7.6 Hz, 2 H, ArH), 7.70 (d, *J =* 6.8 Hz, 2 H, ArH), 7.64 (t, *J =* 7.4 Hz, 1 H, ArH), 7.57-7.48 (m, 3 H, CH₃), 7.43 (t, *J =* 7.2 Hz, 2 H, ArH); **¹³C NMR** (100 MHz, CDCl₃): δ = 177.8, 136.7, 134.0, 132.9, 130.7, 129.4, 128.54, 128.48, 119.9, 93.0, 86.8; **MS** (70 eV, EI) *m*/*z* (%): 206 (M⁺, 65.85), 178 (100); **IR** (neat): v = 2195, 1637, 1597, 1579, 1488, 1447, 1313, 1282, 1207, 1171, 1031, 1010 cm⁻¹.

### Example 13

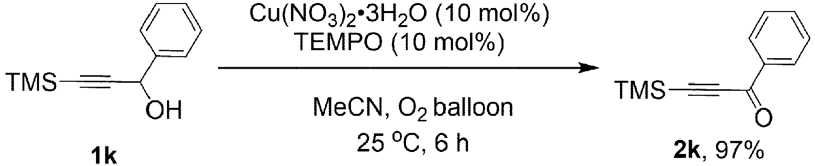

Operations were conducted by referring to Example 1. Cu(NO₃)₂•3H₂O (24.2 mg, 0.1 mmol), TEMPO (15.9 mg, 0.1 mmol), **1k** (204.8 mg, 1.0 mmol), MeCN (4 mL), reacting 6 hours to afford **2k** (197.2 mg, 97%) (eluent: petroleum ether/ethyl ether = 60/1): oily liquid; **¹H NMR** (400 MHz, CDCl₃): δ = 8.15 (d, *J =* 7.2 Hz, 2 H, ArH), 7.62 (t, *J =* 7.4 Hz, 1 H, ArH), 7.50 (t, *J =* 7.6 Hz, 2 H, ArH), 0.33 (s, 9 H, 3×CH₃); **¹³C NMR** (100 MHz, CDCl₃): δ = 177.6, 136.4, 134.1, 129.6, 128.5, 100.8, 100.5, -0.8.

### Example 14

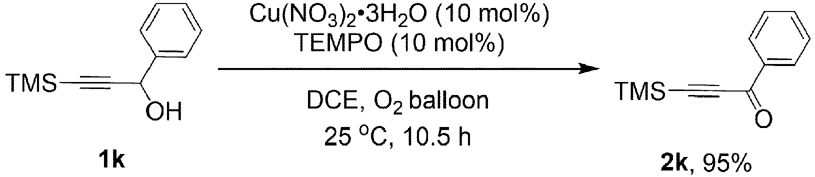

Operations were conducted by referring to Example 1. Cu(NO₃)₂•3H₂O (24.4 mg, 0.1 mmol), TEMPO (15.9 mg, 0.1 mmol), **1k** (204.1 mg, 1.0 mmol), DCE (4 mL), reacted 10.5 hours to afford **2k** (191.5 mg, 95%) (eluent: petroleum ether/ethyl ether = 60/1): oily liquid; **¹H NMR** (400 MHz, CDCl₃): δ = 8.20-8.10 (m, 2 H, ArH), 7.62 (t, *J =* 7.6 Hz, 1 H, ArH), 7.49 (t, *J =* 7.6 Hz, 2 H, ArH), 0.33 (s, 9 H, 3×CH₃); **¹³C NMR** (100 MHz, CDCl₃): δ = 177.6, 136.4, 134.1, 129.6, 128.5, 100.8, 100.5, -0.7.

### Example 15

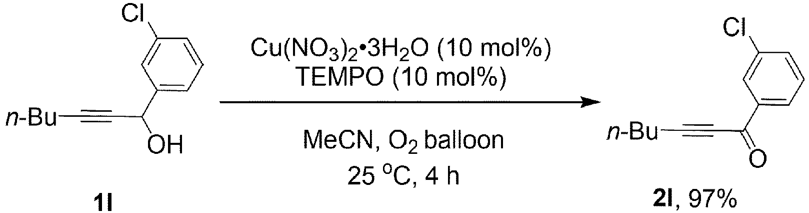

Operations were conducted by referring to Example 1. Cu(NO₃)₂•3H₂O (24.4 mg, 0.1 mmol), TEMPO (16.0 mg, 0.1 mmol), **11** (222.8 mg, 1.0 mmol), MeCN (4 mL), reacted 4 hours to afford **21** (213.9 mg, 97%) (eluent: petroleum ether/ethyl ether = 30/1): oily liquid; **¹H NMR** (400 MHz, CDCl₃): δ = 8.10 (t, *J =* 1.8 Hz, 1 H, ArH), 8.02 (d, *J =* 7.6 Hz, 1 H, ArH), 7.57 (dt, *J*₁ = 7.6 Hz, *J*₁ = 1.2 Hz, 1 H, ArH), 7.43 (t, *J* = 7.8 Hz, 1 H, ArH), 2.52 (t, *J* = 7.0 Hz, 2 H, CH₂), 1.74-1.60 (m, 2 H, CH₂), 1.58-1.44 (m, 2 H, CH₂), 0.97 (t, *J=* 7.4 Hz, 3 H, CH₃); **¹³C NMR** (100 MHz, CDCl₃): δ = 176.6, 138.3, 134.7, 133.6, 129.8, 129.3, 127.5, 97.7, 79.2, 29.6, 22.0, 18.8, 13.4; **MS** (70 eV, EI) *m*/*z* (%): 222 (M⁺ (³⁷Cl), 10.02), 220 (M⁺ (³⁵Cl), 29.95), 139 (100); **IR** (neat): v = 2959, 2933, 2871, 2204, 1645, 1571, 1424, 1286, 1245 cm⁻¹; **HRMS** calcd for C₁₃H₁₃O³⁵Cl [M⁺]: 220.0655, found: 220.0657.

### Example 16

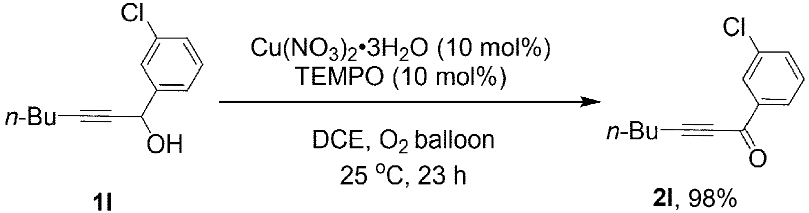

Operations were conducted by referring to Example 1. Cu(NO₃)₂•3H₂O (24.0 mg, 0.1 mmol), TEMPO (15.9 mg, 0.1 mmol), **11** (222.5 mg, 1.0 mmol), DCE (4 mL), reacted 23 hours to afford **21** (216.2 mg, 98%) (eluent: petroleum ether/ethyl ether = 30/1): oily liquid; **¹H NMR** (400 MHz, CDCl₃): δ = 8.10 (t, *J =* 1.8 Hz, 1 H, ArH), 8.02 (d, *J =* 7.6 Hz, 1 H, ArH), 7.60-7.53 (m, 1 H, ArH), 7.42 (t, *J =* 7.8 Hz, 1 H, ArH), 2.52 (t, *J =* 7.0 Hz, 2 H, CH₂), 1.74-1.60 (m, 2 H, CH₂), 1.58-1.44 (m, 2 H, CH₂), 0.98 (t, *J =* 7.4 Hz, 3 H, CH₃); **¹³C NMR** (100 MHz, CDCl₃): δ = 176.6, 138.4, 134.7, 133.6, 129.8, 129.3, 127.5, 97.7, 79.2, 29.7, 22.0, 18.8, 13.4.

### Example 17

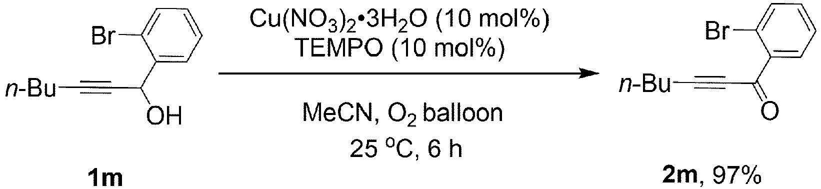

Operations were conducted by referring to Example 1. Cu(NO₃)₂•3H₂O (24.4 mg, 0.1 mmol), TEMPO (16.0 mg, 0.1 mmol), **1m** (266.2 mg, 1.0 mmol), MeCN (4 mL), reacted 6 hours to afford **2m** (257.4 mg, 97%) (eluent: petroleum ether/ethyl ether = 30/1): oily liquid; **¹H NMR** (400 MHz, CDCl₃): δ = 8.00 (d, *J =* 7.6 Hz, 1 H, ArH), 7.67 (d, *J=* 8.0 Hz, 1 H, ArH), 7.47-7.31 (m, 2 H, ArH), 2.48 (t, *J=* 7.0 Hz, 2 H, CH₂), 1.70-1.56 (m, 2 H, CH₂), 1.55-1.41 (m, 2 H, CH₂), 0.95 (t, *J =* 7.4 Hz, 3 H, CH₃); **¹³C NMR** (100 MHz, CDCl₃): δ = 177.5, 137.4, 134.8, 133.0, 132.7, 127.2, 120.9, 98.0, 80.6, 29.5, 22.0, 18.9, 13.4; **MS** (70 eV, EI) *m*/*z* (%): 266 (M⁺ (⁸¹Br), 9.56), 264 (M⁺ (⁷⁹Br), 9.29), 185 (100); **IR** (neat): v = 2187, 1648, 1580, 1474, 1386, 1262, 1063, 1008 cm⁻¹.

### Example 18

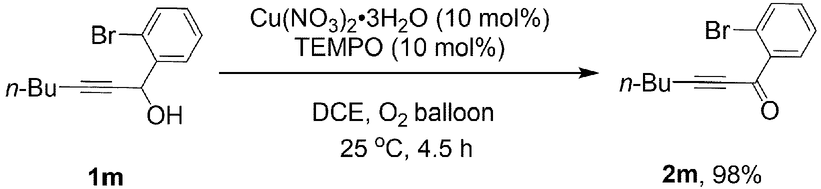

Operations were conducted by referring to Example 1. Cu(NO₃)₂•3H₂O (24.4 mg, 0.1 mmol), TEMPO (16.0 mg, 0.1 mmol), **1m** (267.2 mg, 1.0 mmol), DCE (4 mL), reacted 4.5 hours to afford **2m** (260.6 mg, 98%) (eluent: petroleum ether/ethyl ether = 30/1): oily liquid; **¹H NMR** (400 MHz, CDCl₃): δ = 8.00 (dd, *J*₁ = 8.0 Hz, *J*₂ = 1.6 Hz, 1 H, ArH), 7.67 (d, *J* = 8.0 Hz, 1 H, ArH), 7.42 (td, *J*₁ = 7.2 Hz, *J*₂ = 1.2 Hz, 1 H, ArH), 7.35 (td, *J*₁ = 7.6 Hz, *J*₂ = 1.6 Hz, 1 H, ArH), 2.48 (t, *J =* 7.0 Hz, 2 H, CH₂), 1.70-1.56 (m, 2 H, CH₂), 1.55-1.41 (m, 2 H, CH₂), 0.95 (t*, J =* 7.2 Hz, 3 H, CH₃); **¹³C NMR** (100 MHz, CDCl₃): δ = 177.5, 137.3, 134.7, 133.0, 132.7, 127.1, 120.8, 97.9, 80.6, 29.5, 21.9, 18.9, 13.4.

### Example 19

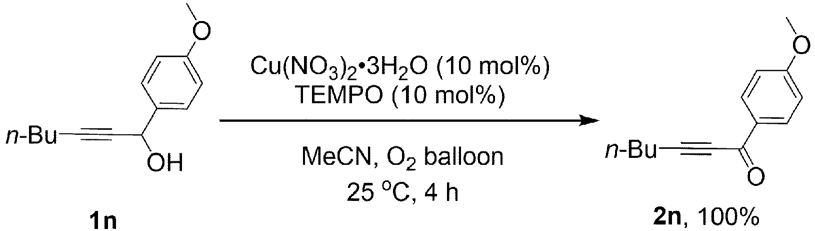

Operations were conducted by referring to Example 1. Cu(NO₃)₂•3H₂O (24.4 mg, 0.1 mmol), TEMPO (16.0 mg, 0.1 mmol), **In** (222.8 mg, 1.0 mmol), MeCN (4 mL), reacted 4 hours to afford **2n** (214.3 mg, 100%) (eluent: petroleum ether/ethyl ether = 20/1): oily liquid; **¹H NMR** (400 MHz, CDCl₃): δ = 8.11 (d, *J =* 8.4 Hz, 2 H, ArH), 6.95 (d, *J* = 8.4 Hz, 2 H, ArH), 3.89 (s, 3 H, CH₃), 2.49 (t, *J* = 7.2 Hz, 2 H, CH₂), 1.72-1.56 (m, 2 H, CH₂), 1.54-1.46 (m, 2 H, CH₂), 0.97 (t, *J =* 7.2 Hz, 3 H, CH₃); **¹³C NMR** (100 MHz, CDCl₃): δ = 176.8, 164.2, 131.8, 130.2, 113.6, 95.8, 79.5, 55.4, 29.8, 22.0, 18.7, 13.4.

### Example 20

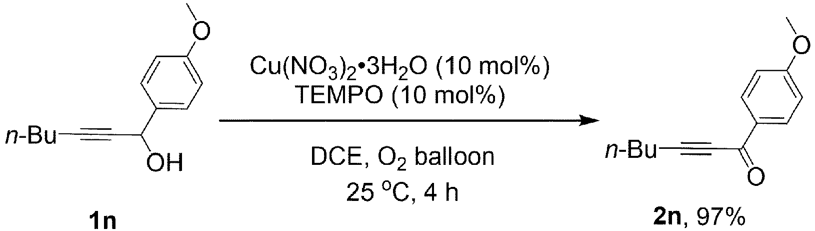

Operations were conducted by referring to Example 1. Cu(NO₃)₂•3H₂O (24.4 mg, 0.1 mmol), TEMPO (16.0 mg, 0.1 mmol), **In** (218.5 mg, 1.0 mmol), DCE (4 mL), reacted 4 hours to afford **2n** (209.6 mg, 97%) (eluent: petroleum ether/ethyl ether = 30/1(300 mL), petroleum ether/ethyl ether = 15/1): oily liquid; **¹H NMR** (400 MHz, CDCl₃): δ = 8.11 (d, *J =* 8.8 Hz, 2 H, ArH), 6.95 (d, *J =* 8.8 Hz, 2 H, ArH), 3.89 (s, 3 H, CH₃), 2.49 (t, *J =* 7.6 Hz, 2 H, CH₂), 1.72-1.58 (m, 2 H, CH₂), 1.56-1.44 (m, 2 H, CH₂), 0.96 (t, *J =* 7.4 Hz, 3 H, CH₃); **¹³C NMR** (100 MHz, CDCl₃): δ = 176.9, 164.2, 131.8, 130.2, 113.6, 95.8, 79.5, 55.4, 29.8, 22.0, 18.7, 13.4.

### Example 21

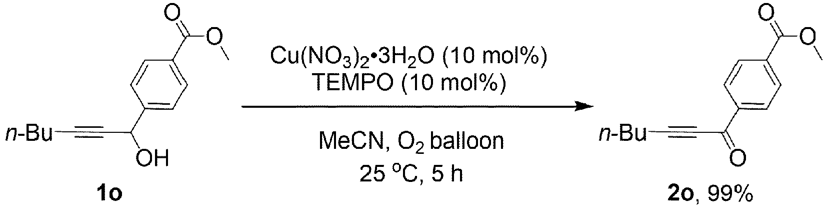

Operations were conducted by referring to Example 1. Cu(NO₃)₂•3H₂O (24.2 mg, 0.1 mmol), TEMPO (15.9 mg, 0.1 mmol), **1o** (246.3 mg, 1.0 mmol), MeCN (4 mL), reacted 5 hours to afford **2o** (243.0 mg, 99%) (eluent: petroleum ether/ethyl ether = 20/1): oily liquid; **¹H NMR** (400 MHz, CDCl₃): δ = 8.19 (d, *J =* 8.8 Hz, 2 H, ArH), 8.13 (d, *J =* 8.8 Hz, 2 H, ArH), 3.96 (s, 3 H, CH₃), 2.53 (t, *J =* 7.2 Hz, 2 H, CH₂), 1.73-1.62 (m, 2 H, CH₂), 1.58-1.45 (m, 2 H, CH₂), 0.98 (t, *J =* 7.4 Hz, 3 H, CH₃); **¹³C NMR** (100 MHz, CDCl₃): δ = 177.1, 165.9, 139.8, 134.3, 129.5, 129.2, 97.9, 79.4, 52.3, 29.6, 21.9, 18.8, 13.3; **MS** (70 eV, EI) *m*/*z* (%): 244 (M⁺, 14.91), 202 (100); **IR** (neat): v = 2956, 2933, 2872, 2237, 2198, 1724, 1646, 1435, 1407, 1276, 1259, 1244, 1116, 1102, 1017 cm⁻¹; **HRMS** calcd. for C₁₅H₁₆O₃ (M⁺): 244.1099; Found: 244.1099.

### Example 22

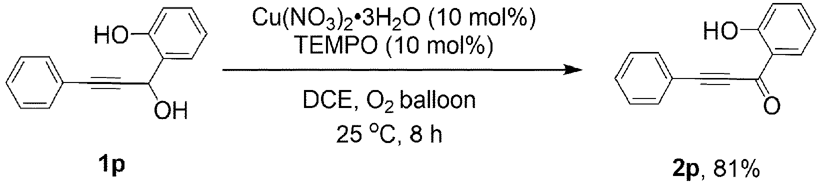

Operations were conducted by referring to Example 1. Cu(NO₃)₂•3H₂O (24.2 mg, 0.1 mmol), TEMPO (16.0 mg, 0.1 mmol), **1p** (224.2 mg, 1.0 mmol), DCE (4 mL), reacted 8 hours to afford **2p** (180.1 mg, 81%) (eluent: petroleum ether/ethyl ether = 60/1): yellow solid. Melting point: 64.0-65.6 °C (petroleum ether/ethyl acetate recrystallization); **¹H NMR** (400 MHz, CDCl₃): δ = 11.75 (s, 1 H, OH), 8.13 (d, *J =* 8.4 Hz, 1 H, ArH), 7.76-7.64 (m, 2 H, ArH), 7.58-7.38 (m, 4 H, ArH), 7.00 (t, *J =* 80 Hz, 2 H, ArH); **¹³C NMR** (100 MHz, CDCl₃): δ = 182.2, 162.7, 137.1, 133.1, 133.0, 131.1, 128.7, 120.7, 119.6, 119.4, 118.1, 96.0, 85.6.

### Example 23

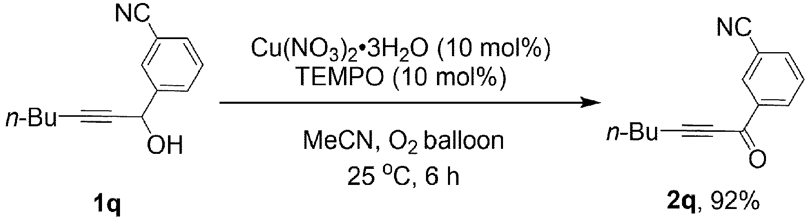

Operations were conducted by referring to Example 1. Cu(NO₃)₂•3H₂O (24.1 mg, 0.1 mmol), TEMPO (16.0 mg, 0.1 mmol), **1q** (213.0 mg, 1.0 mmol), MeCN (4 mL), reacted 6 hours to afford **2q** (194.8 mg, 92%) (eluent: petroleum ether/ethyl ether = 20/1): oily liquid; **¹H NMR** (400 MHz, CDCl₃): δ = 8.42 (s, 1 H, ArH), 8.34 (dd, *J*₁ *=* 8.0 Hz, *J*₂ = 1.2 Hz, 1 H, ArH), 7.87 (d, *J=* 7.6 Hz, 1 H, ArH), 7.63 (t, *J=* 7.8 Hz, 1 H, ArH), 2.55 (t, *J =* 7.0 Hz, 2 H, CH₂), 1.75-1.63 (m, 2 H, CH₂), 1.62-1.45 (m, 2 H, CH₂), 0.98 (t, *J =* 7.2 Hz, 3 H, CH₃); **¹³C NMR** (100 MHz, CDCl₃): δ = 175.6, 137.4, 136.5, 133.1, 132.9, 129.5, 117.7, 112.9, 98.9, 78.8, 29.5, 21.9, 18.8, 13.3; **MS** (70 eV, EI) *m*/*z* (%): 211 (M⁺, 7.31), 169 (100); **IR** (neat): v = 2959, 2933, 2872, 2219, 2200, 1647, 1598, 1579, 1465, 1427, 1292, 1265, 1180 cm⁻¹; **HRMS** calcd. for C₁₄H₁₃NO (M⁺): 211.0997; Found: 211.0993.

### Example 24

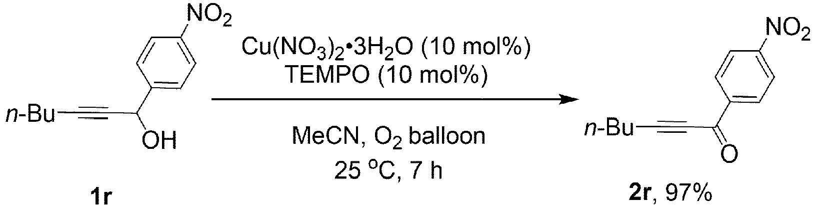

Operations were conducted by referring to Example 1. Cu(NO₃)₂•3H₂O (24.4 mg, 0.1 mmol), TEMPO (15.7 mg, 0.1 mmol), **1r** (233.3 mg, 1.0 mmol), MeCN (4 mL), reacted 7 hours to afford **2r** (223.7 mg, 97%) (eluent: petroleum ether/ethyl ether = 40/1): oily liquid; **¹H NMR** (400 MHz, CDCl₃): δ = 8.33 (d, *J* = 9.2 Hz, 2 H, ArH), 8.29 (d, *J =* 9.2 Hz, 2 H, ArH), 2.55 (t, *J =* 70 Hz, 2 H, CH₂), 1.73-1.64 (m, 2 H, CH₂), 1.56-1.46 (m, 2 H, CH₂), 0.98 (t, *J=* 7.2 Hz, 3 H, CH₃); **¹³C NMR** (100 MHz, CDCl₃): δ = 175.8, 150.6, 140.9, 130.2, 123.5, 99.3, 79.2, 29.5, 21.9, 18.8, 13.3; **MS** (70 eV, EI) *m*/*z* (%): 231 (M⁺, 4.21), 189 (100); **IR** (neat): v = 2959, 2934, 2871, 2237, 2199, 1650, 1602, 1524, 1343, 1320, 1257, 1104 cm⁻¹.

### Example 25

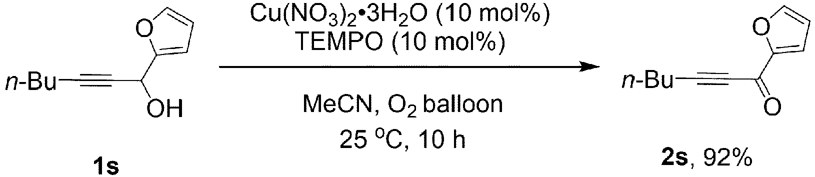

Operations were conducted by referring to Example 1. Cu(NO₃)₂•3H₂O (24.2 mg, 0.1 mmol), TEMPO (16.0 mg, 0.1 mmol), **1s** (177.8 mg, 1.0 mmol), MeCN (4 mL), reacted 10 hours to afford **2s** (162.5 mg, 92%) (eluent: petroleum ether/ethyl ether = 10/1): oily liquid; **¹H NMR** (400 MHz, CDCl₃): δ = 7.65 (s, 1 H, H from furyl), 7.32 (d, *J* = 3.2 Hz, 1 H, H from furyl), 6.56 (dd, *J*₁ = 3.6 Hz, *J*₂ = 1.6 Hz, 1 H, H from furyl), 2.47 (t, *J =* 7.0 Hz, 2 H, CH₂), 1.74-1.58 (m, 2 H, CH₂), 1.56-1.42 (m, 2 H, CH₂), 0.96 (t, *J =* 7.2 Hz, 3 H, CH₃); **¹³C NMR** (100 MHz, CDCl₃): δ = 164.9, 153.1, 147.6, 120.6, 112.4, 95.4, 78.8, 29.5, 21.8, 18.6, 13.3; **MS** (70 eV, EI) *m*/*z* (%): 176 (M⁺, 27.69), 95 (100); **IR** (neat): v = 2957, 2933, 2868, 2251, 2207, 1631, 1562, 1460, 1390, 1294, 1168, 1123, 1014 cm⁻¹.

### Example 26

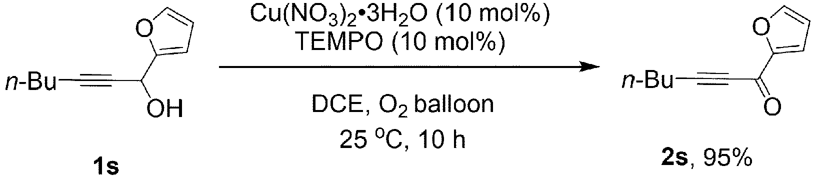

Operations were conducted by referring to Example 1. Cu(NO₃)₂•3H₂O (24.0 mg, 0.1 mmol), TEMPO (16.0 mg, 0.1 mmol), **1s** (178.5 mg, 1.0 mmol), DCE(4mL), reacted 10 hours to afford **2s** (168.0 mg, 95%) (eluent: petroleum ether/ethyl ether = 15/1): oily liquid; **¹H NMR** (400 MHz, CDCl₃): δ = 7.64 (s, 1 H, H from furyl), 7.31 (d, *J =* 3.2 Hz, 1 H, H from furyl), 6.56 (t, *J =* 1.6 Hz, 1 H, H from furyl), 2.47 (t, *J =* 7.0 Hz, 2 H, CH₂), 1.70-1.56 (m, 2 H, CH₂), 1.56-1.43 (m, 2 H, CH₂), 0.96 (t, *J =* 7.2 Hz, 3 H, CH₃); **¹³C NMR** (100 MHz, CDCl₃): δ = 164.4, 152.7, 147.2, 120.1, 111.9, 94.9, 78.4, 29.1, 21.4, 18.2, 12.9.

### Example 27

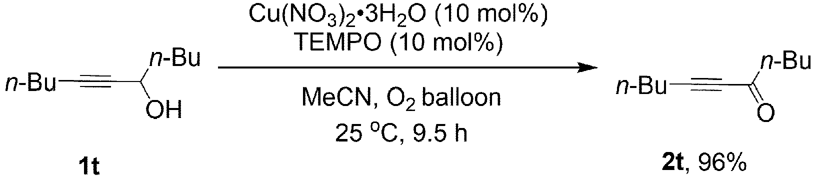

Operations were conducted by referring to Example 1. Cu(NO₃)₂•3H₂O (23.8 mg, 0.1 mmol), TEMPO (15.9 mg, 0.1 mmol), **It** (169.1 mg, 1.0 mmol), MeCN (4 mL), reacted 9.5 hours to afford **2t** (160.1 mg, 96%) (eluent: petroleum ether/ethyl ether = 60/1): oily liquid; **¹H NMR** (400 MHz, CDCl₃): δ = 2.53 (t, *J =* 7.4 Hz, 2 H, CH₂), 2.37 (t, *J =* 7.0 Hz, 2 H, CH₂), 1.70-1.50 (m, 4 H, CH₂CH₂), 1.50-1.25 (m, 4 H, CH2CH2), 1.00-0.86 (m, 6 H, 2×CH₃); **¹³C NMR** (100 MHz, CDCl₃): δ = 188.3, 94.0, 80.7, 45.1, 29.6, 26.1, 22.0, 21.8, 18.4, 13.6, 13.3.

### Example 28

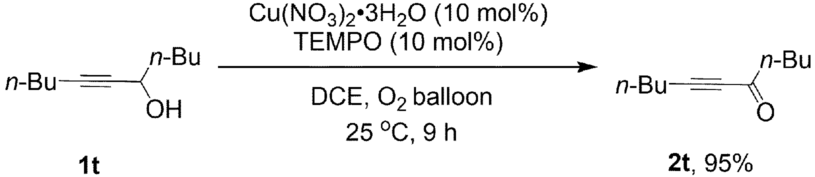

Operations were conducted by referring to Example 1. Cu(NO₃)₂•3H₂O (24.0 mg, 0.1 mmol), TEMPO (16.0 mg, 0.1 mmol), **It** (168.6 mg, 1.0 mmol), DCE(4mL), reacted 9 hours to afford **2t** (158.8 mg, 95%) (eluent: petroleum ether/ethyl ether = 40/1): oily liquid; **¹H NMR** (400 MHz, CDCl₃): δ = 2.53 (t, *J =* 7.4 Hz, 2 H, CH₂), 2.37 (t, *J* = 7.0 Hz, 2 H, CH₂), 1.70-1.50 (m, 4 H, CH₂-CH₂), 1.50-1.17 (m, 4 H, CH₂-CH₂), 0.98-0.88 (m, 6 H, 2×CH₃); **¹³C NMR** (100 MHz, CDCl₃): δ = 188.4, 94.1, 80.8, 45.2, 29.7, 26.1, 22.0, 21.9, 18.5, 13.7, 13.4.

### Example 29

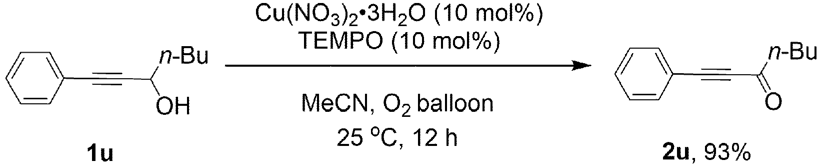

Operations were conducted by referring to Example 1. Cu(NO₃)₂•3H₂O (24.0 mg, 0.1 mmol), TEMPO (15.9 mg, 0.1 mmol), **1u** (188.4 mg, 1.0 mmol), MeCN (4 mL), reacted 12 hours to afford **2u** (173.2 mg, 93%) (eluent: petroleum ether/ethyl ether = 30/1): oily liquid; **¹H NMR** (400 MHz, CDCl₃): δ = 7.58 (d, *J =* 7.2 Hz, 2 H, ArH), 7.50-7.34 (m, 3 H, ArH), 2.67 (t, *J =* 7.2 Hz, 2 H, CH₂), 1.80-1.66 (m, 2 H, CH₂), 1.48-1.32 (m, 2 H, CH₂), 0.95 (t, *J =* 7.4 Hz, 3 H, CH₃); **¹³C NMR** (100 MHz, CDCl₃): δ = 188.2, 132.9, 130.5, 128.5, 120.0, 90.4, 87.8, 45.1, 26.1, 22.1, 13.7; **MS** (70 eV, EI) *m*/*z* (%): 186 (M⁺, 1.30), 129 (100); **IR** (neat): v = 2958, 2932, 2872, 2200, 1666, 1489, 1272, 1125, 1067 cm⁻¹.

### Example 30

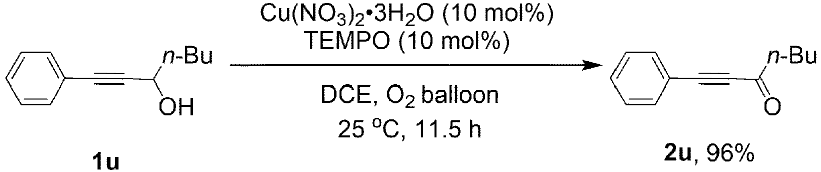

Operations were conducted by referring to Example 1. Cu(NO₃)₂•3H₂O (24.2 mg, 0.1 mmol), TEMPO (16.2 mg, 0.1 mmol), **1u** (188.4 mg, 1.0 mmol), DCE(4mL), reacted 11.5 hours to afford **2u** (179.8 mg, 96%) (eluent: petroleum ether/ethyl ether = 30/1): oily liquid; **¹H NMR** (400 MHz, CDCl₃): δ = 7.61-7.54 (m, 2 H, ArH), 7.49-7.35 (m, 3 H, ArH), 2.67 (t, *J =* 7.4 Hz, 2 H, CH₂), 1.80-1.66 (m, 2 H, CH₂), 1.47-1.34 (m, 2 H, CH₂), 0.95 (t, *J =* 7.2 Hz, 3 H, CH₃); **¹³C NMR** (100 MHz, CDCl₃): δ = 187.9, 132.8, 130.4, 128.4, 119.9, 90.3, 87.7, 45.0, 26.0, 21.9, 13.6.

### Example 31

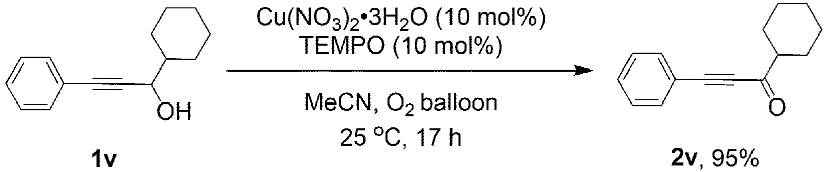

Operations were conducted by referring to Example 1. Cu(NO₃)₂•3H₂O (24.1 mg, 0.1 mmol), TEMPO (16.0 mg, 0.1 mmol), **1v** (214.1 mg, 1.0 mmol), MeCN (4 mL), reacted 17 hours to afford **2v** (200.7 mg, 95%) (eluent: petroleum ether/ethyl ether = 30/1): oily liquid; **¹H NMR** (400 MHz, CDCl₃): δ = 7.59 (d, *J =* 7.2 Hz, 2 H, ArH), 7.50-7.34 (m, 3 H, ArH), 2.57-2.44 (m, 1 H, CH), 2.06 (d, *J* = 11.2 Hz, 2 H, CH₂), 1.82 (dd, *J*₁ = 9.2 Hz, *J*₂ = 3.6 Hz, 2 H, CH₂), 1.69 (d, *J=* 12.0 Hz, 1 H, one proton of CH₂), 1.50 (dd, *J*₁ = 23.2 Hz, *J*₂ = 11.4 Hz, 2 H, CH₂), 1.42-1.16 (m, 3 H, 3 H from Cy); **¹³C NMR** (100 MHz, CDCl₃): δ = 191.3, 132.9, 130.5, 128.5, 120.1, 91.2, 87.1, 52.2, 28.2, 25.7, 25.3; **MS** (70 eV, EI) *m*/*z* (%): 212 (M⁺, 4.97), 129 (100); **IR** (neat): v = 2929, 2853, 2196, 1660, 1488, 1445, 1262, 1142, 1089, 1069 cm⁻¹.

### 32

### Example 32

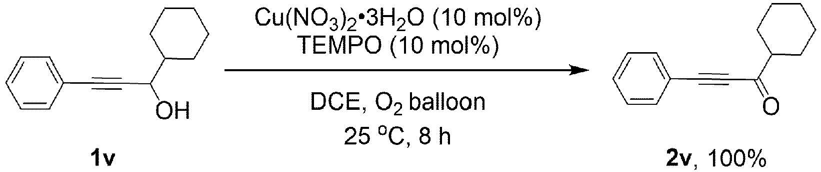

Operations were conducted by referring to Example 1. Cu(NO₃)₂•3H₂O (24.0 mg, 0.1 mmol), TEMPO (16.0 mg, 0.1 mmol), **1v** (214.2 mg, 1.0 mmol), DCE(4mL), reacted 8 hours to afford **2v** (211.4 mg, 100%) (eluent: petroleum ether/ethyl ether = 30/1): oily liquid; **¹H NMR** (400 MHz, CDCl₃): δ = 7.58 (d, *J =* 7.2 Hz, 2 H, ArH), 7.50-7.34 (m, 3 H, ArH), 2.57-2.44 (m, 1 H, CH), 2.06 (d, *J =* 10.8 Hz, 2 H, CH₂), 1.89-1.76 (m, 2 H, CH₂), 1.74-1.16 (m, 6 H, CH₂CH₂CH₂); **¹³C NMR** (100 MHz, CDCl₃): δ = 191.3, 132.9, 130.4, 128.5, 120.1, 91.2, 87.1, 52.2, 28.2, 25.7, 25.3.

### Example 33

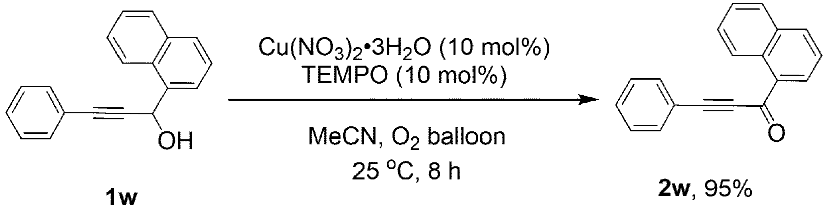

Operations were conducted by referring to Example 1. Cu(NO₃)₂•3H₂O (24.0 mg, 0.1 mmol), TEMPO (16.0 mg, 0.1 mmol), **1w** (258.3 mg, 1.0 mmol), MeCN (4 mL), reacted 8 hours to afford **2w** (244.7 mg, 95%) (eluent: petroleum ether/ethyl ether = 60/1): white solid. Melting point: 93.0-94.7 °C (petroleum ether/ethyl acetate recrystallization); **¹H NMR** (400 MHz, CDCl₃): δ = 9.24 (d, *J =* 8.8 Hz, 1 H, ArH), 8.65 (d, *J =* 6.8 Hz, 1 H, ArH), 8.10 (d, *J =* 80 Hz, 1 H, ArH), 7.92 (d, *J =* 80 Hz, 1 H, ArH), 7.80-7.38 (m, 8 H, ArH); **¹³C NMR** (100 MHz, CDCl₃): δ = 179.6, 135.0, 134.5, 133.8, 132.9, 130.6, 130.5, 128.9, 128.6, 128.5, 126.7, 125.9, 124.4, 120.2, 91.6, 88.4; **MS** (70 eV, EI) *m*/*z* (%):257 (M⁺+1, 16.50), 256 (M⁺, 86.95), 255 (100); **IR** (neat): v = 2192, 1629, 1589, 1570, 1508, 1285, 1176, 1100, 1072 cm⁻¹.

### Example 34

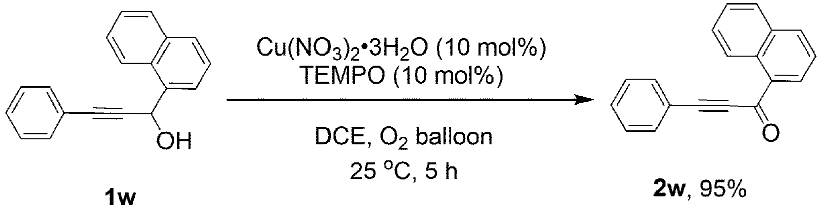

Operations were conducted by referring to Example 1. Cu(NO₃)₂•3H₂O (24.3 mg, 0.1 mmol), TEMPO (15.9 mg, 0.1 mmol), **1w** (258.8 mg, 1.0 mmol), DCE (4 mL), reacted 5 hours to afford **2w** (243.2 mg, 95%) (eluent: petroleum ether/ethyl ether = 40/1): white solid. **¹H NMR** (400 MHz, CDCl₃): δ = 9.24 (d, *J =* 8.8 Hz, 1 H, ArH), 8.65 (d, *J =* 7.2 Hz, 1 H, ArH), 8.09 (d, *J =* 8.4 Hz, 1 H, ArH), 7.91 (d, *J =* 80 Hz, 1 H, ArH), 7.76-7.38 (m, 8 H, ArH); **¹³C NMR** (100 MHz, CDCl₃): δ = 179.6, 135.0, 134.5, 133.8, 132.85, 132.82, 130.6, 130.5, 128.9, 128.6, 128.5, 126.7, 125.9, 124.4, 120.2, 91.6, 88.4.

### Example 35

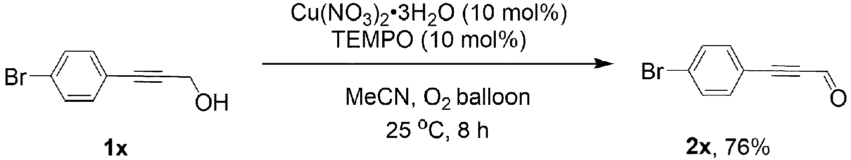

Operations were conducted by referring to Example 1. Cu(NO₃)₂•3H₂O (23.7 mg, 0.1 mmol), TEMPO (16.0 mg, 0.1 mmol), **1x** (211.0 mg, 1.0 mmol), MeCN (4 mL), reacted 8 hours to afford **2x** (159.0 mg, 76%) (eluent: petroleum ether/ethyl ether = 60/1): red solid. Melting point: 92.0-93.4 °C (petroleum ether/ethyl acetate recrystallization); **¹H NMR** (400 MHz, CDCl₃): δ = 9.42 (s, 1 H, CHO), 7.56 (d, *J =* 8.8 Hz, 2 H, ArH), 7.47 (d, *J =* 8.0 Hz, 2 H, ArH); **¹³C NMR** (100 MHz, CDCl₃): δ = 176.5, 134.4, 132.1, 126.2, 118.3, 93.5, 89.0; **MS** (70 eV, EI) *m*/*z* (%): 210 (M⁺(⁸¹Br), 86.41), 208 (M⁺(⁷⁹Br), 86.47), 101 (100); **IR** (neat): v = 2949, 2866, 2203, 1649, 1578, 1460, 1427, 1284, 1238, 1030 cm⁻¹.

### Example 36

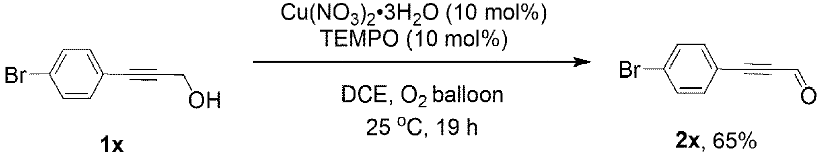

Operations were conducted by referring to Example 1. Cu(NO₃)₂•3H₂O (24.2 mg, 0.1 mmol), TEMPO (16.0 mg, 0.1 mmol), **1x** (211.2 mg, 1.0 mmol), DCE(4mL), reacted 19 hours to afford **2x** (135.8 mg, 65%) (eluent: petroleum ether/ethyl ether = 60/1): red solid. **¹H NMR** (400 MHz, CDCl₃): δ = 9.42 (s, 1 H, CHO), 7.56 (d, *J=* 8.0 Hz, 2 H, ArH), 7.46 (d, *J* = 8.0 Hz, 2 H, ArH); **¹³C NMR** (100 MHz, CDCl₃): δ = 176.5, 134.5, 132.1, 126.2, 118.3, 93.5, 89.0.

### Example 37

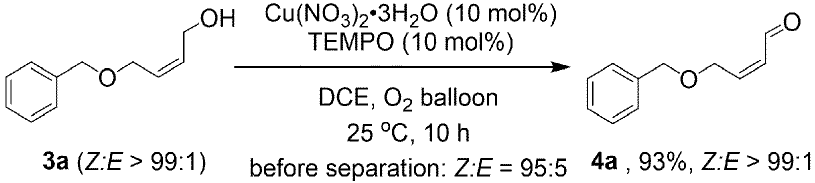

Operations were conducted by referring to Example 1. Cu(NO₃)₂•3H₂O (24.2 mg, 0.1 mmol), TEMPO (16.0 mg, 0.1 mmol), **3a** (187.6 mg, 1.0 mmol), DCE(4mL), reacted 10 hours to afford **4a** (164.4 mg, 93%, *Z:E* > 99:1) (eluent: petroleum ether/ethyl ether = 20/1) (before separation afforded a crude mixture Z:E = 95:5, as determined by ¹H NMR analysis): oily liquid; **¹H NMR** (400 MHz, CDCl₃): 10.05 (d, *J=* 6.8 Hz, 1 H, CHO), 7.42-7.28 (m, 5 H, ArH), 6.64 (dt, *J*₁ = 11.2 Hz, *J*₂ = 5.6 Hz, 1 H, CH=), 6.07 (ddt, *J*₁ = 11.2 Hz, *J*₂ = 6.4 Hz, *J*₃ = 2.0 Hz, 1 H, CH=), 4.59 (s, 2 H, ArCH₂), 4.53 (dd, *J*₁ = 5.6 Hz, *J*₂ = 2.0 Hz, 2 H, OCH₂); **¹³C NMR** (100 MHz, CDCl₃): δ = 191.4, 147.5, 137.2, 129.6, 128.5, 127.9, 127.7, 73.0, 66.9.

### Example 38

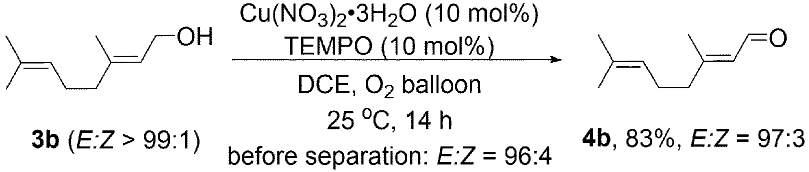

Operations were conducted by referring to Example 1. Cu(NO₃)₂•3H₂O (24.4 mg, 0.1 mmol), TEMPO (16.0 mg, 0.1 mmol), **3b** (155.4 mg, 1.0 mmol), DCE (10 mL), reacted 14 hours to afford **4b** (126.3 mg, 83%, E:Z = 97:3) (eluent: petroleum ether/ethyl ether = 50/1(400 mL), petroleum ether/ethyl ether = 20/1(300 mL)) (before separation afforded a crude mixture *E:Z* = 96:4, as determined by ¹H NMR analysis): oily liquid; **¹H NMR** (400 MHz, CDCl₃): δ = 10.00 (d, *J =* 8.0 Hz, 1 H, CHO), 5.88 (d, *J =* 8.0 Hz, 1 H, CH=), 5.08 (d, *J =* 6.4 Hz, 1 H, CH=), 2.30-2.16 (m, 4 H, 2xCH₂), 2.17 (s, 3 H, CH₃), 1.69 (s, 3 H, CH₃), 1.61 (s, 3 H, CH₃); **¹³C NMR** (100 MHz, CDCl₃): δ = 191.1, 163.6, 132.7, 127.2, 122.3, 40.4, 25.5, 25.4, 17.5, 17.3.

### Example 39

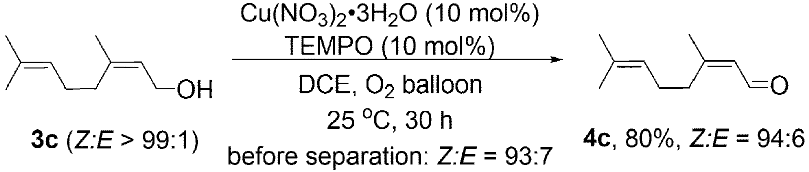

Operations were conducted by referring to Example 1. Cu(NO₃)₂•3H₂O (24.6 mg, 0.1 mmol), TEMPO (16.1 mg, 0.1 mmol), **3c** (153.6 mg, 1.0 mmol), DCE (10 mL), reacted 30 hours to afford **4c** (118.1 mg, 80%, *Z:E* = 94:6) (eluent: petroleum ether/ethyl ether = 15/1) (before separation afforded a crude mixture Z:E = 93:7, as determined by ¹H NMR analysis): oily liquid; **¹H NMR** (400 MHz, CDCl₃):6 = 9.90 (d, *J =* 8.4 Hz, 1 H, CHO), 5.88 (d, *J =* 8.4 Hz, 1 H, CH=), 5.10 (t, *J =* 7.0 Hz, 1 H, CH=), 2.59 (t, *J* = 7.6 Hz, 2 H, CH₂), 2.24 (dd, *J*₁ = 14.6 Hz, *J*₂ = 7.4 Hz, 2 H, CH₂), 1.99 (s, 3 H, CH₃), 1.69 (s, 3 H, CH₃), 1.61 (s, 3 H, CH₃); **¹³C NMR** (100 MHz, CDCl₃): δ = 190.2, 163.3, 133.1, 128.1, 121.8, 32.1, 26.5, 25.1, 24.5, 17.2; the following signals are discernible for *E***-4c:** δ = 190.8, 132.4, 126.9, 122.1, 40.1, 25.2, 17.1.

### Example 40

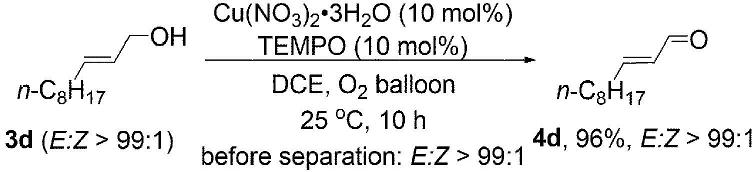

Operations were conducted by referring to Example 1. Cu(NO₃)₂•3H₂O (24.2 mg, 0.1 mmol), TEMPO (16.0 mg, 0.1 mmol), **3d** (177.3 mg, 1.0 mmol), DCE (4 mL), reacted 10 hours to afford **4d** (161.3 mg, 96%, *E:Z* > 99:1) (eluent: petroleum ether/ethyl ether = 50/1) (before separation afforded a crude mixture E:Z > 99:1, as determined by ¹H NMR analysis): oily liquid; ¹**H NMR** (400 MHz, CDCl₃): δ = 9.51 (d, *J =* 8.0 Hz, 1 H, CHO), 6.86 (dt, *J*₁ = 15.8 Hz, *J*₂ = 7.0 Hz, 1 H, CH=), 6.18-6.07 (m, 1 H, CH=), 2.38-2.27 (m, 2 H, CH₂), 1.56-1.44 (m, 2 H, CH₂), 1.40-1.20 (m, 10 H, 5×CH2), 0.89 (t, *J* = 6.8 Hz, 3 H, CH3); **¹³C NMR** (100 MHz, CDCl₃): δ = 194.0, 158.9, 132.9, 32.6, 31.7, 29.2, 29.06, 29.04, 27.7, 22.5, 14.0.

### Example 41

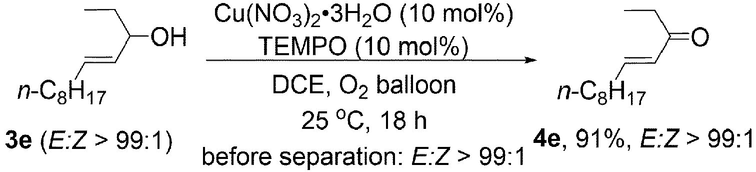

Operations were conducted by referring to Example 1. Cu(NO₃)₂•3H₂O (24.2 mg, 0.1 mmol), TEMPO (16.0 mg, 0.1 mmol), **3e** (199.4 mg, 1.0 mmol), DCE(4mL), reacted 18 hours to afford **4e** (178.9 mg, 91%) (eluent: petroleum ether/ethyl ether = 100/1) (before separation afforded a crude mixture E:Z > 99:1, as determined by ¹H NMR analysis): oily liquid; ¹**H NMR** (400 MHz, CDCl₃): δ = 6.83 (dt, *J*₁ = 16.0 Hz, *J*₂ = 7.0 Hz, 1 H, =CH), 6.09 (d, *J* = 16.0 Hz, 1 H, =CH), 2.56 (q, *J =* 7.4 Hz, 2 H, CH2), 2.20 (q, *J =* 7.2 Hz, 2 H, CH2), 1.56-1.18 (m, 12 H, 6×CH₂), 1.10 (t, *J =* 7.4 Hz, 3 H, CH₃), 0.88 (t, *J =* 6.6 Hz, 3 H, CH₃); **¹³C NMR** (100 MHz, CDCl₃): δ = 201.0, 147.0, 129.9, 33.0, 32.3, 31.7, 29.2, 29.0, 28.0, 22.5, 13.9, 8.0; **MS** (70 eV, EI) *m*/*z* (%): 196 (M⁺, 2.27), 167 (100); **IR** (neat): v = 2925, 2855, 1699, 1675, 1630, 1460, 1355, 1200, 1116 cm⁻¹; **HRMS** calcd. for C₁₃H₂₄O (M⁺): 196.1827; Found: 196.1830.

### Example 42

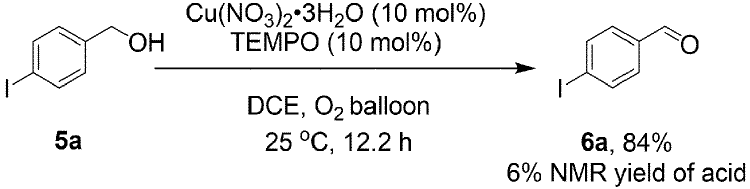

Operations were conducted by referring to Example 1. Cu(NO₃)₂•3H₂O (24.1 mg, 0.1 mmol), TEMPO (16.0 mg, 0.1 mmol), **5a** (241.5 mg, 1.0 mmol), DCE(4mL), reacted 12.2 hours to afford **6a** (194.5 mg, 84%) (eluent: petroleum ether/ethyl ether = 30/1): white solid. **¹H NMR** (400 MHz, CDCl₃): δ = 9.96 (s, 1 H, CHO), 7.92 (d, *J =* 8.0 Hz, 2 H, ArH), 7.60 (d, *J =* 8.0 Hz, 2 H, ArH); **¹³C NMR** (100 MHz, CDCl₃): δ = 191.4, 138.4, 135.5, 130.8, 102.8.

### Example 43

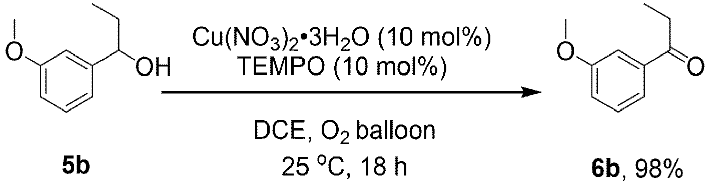

Operations were conducted by referring to Example 1. Cu(NO₃)₂•3H₂O (24.0 mg, 0.1 mmol), TEMPO (16.0 mg, 0.1 mmol), **5b** (165.8 mg, 1.0 mmol), DCE(4mL), reacted 18 hours to afford **6b** (159.8 mg, 98%) (eluent: petroleum ether/ethyl ether = 20/1): oily liquid; **¹H NMR** (400 MHz, CDCl₃): δ = 7.54 (d, *J =* 7.6 Hz, 1 H, ArH), 7.50 (s, 1 H, ArH), 7.36 (t, *J =* 7.8 Hz, 1 H, ArH), 7.10 (dd, *J*₁ = 8.4 Hz, *J*₂ = 2.4 Hz, 1 H, ArH), 3.86 (s, 3 H, CH₃), 2.99 (q, *J =* 7.4 Hz, 2 H, CH₂), 1.22 (t, *J =* 7.2 Hz, 3 H, CH₃); **¹³C NMR** (100 MHz, CDCl₃): δ = 200.1, 159.3, 137.8, 129.0, 120.1, 118.7, 111.8, 54.9, 31.4, 7.7; **MS** (70 eV, EI) *m*/*z* (%): 165 (M⁺+1, 3.73), 164 (M⁺, 35.35), 135 (100); **IR** (neat): v = 2976, 2938, 1686, 1582, 1485, 1461, 1429, 1286, 1254, 1197, 1171, 1044, 1020 cm⁻¹.

### Example 44

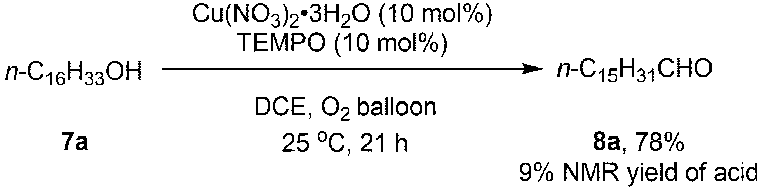

Operations were conducted by referring to Example 1. Cu(NO₃)₂•3H₂O (24.1 mg, 0.1 mmol), TEMPO (16.0 mg, 0.1 mmol), **7a** (247.4 mg, 1.0 mmol), DCE (4 mL), reacted 21 hours to afford **8a** (187.8 mg, 78%) (eluent: petroleum ether/ethyl ether = 60/1): white solid. **¹H NMR** (400 MHz, CDCl₃): δ = 9.77 (t, *J =* 1.6 Hz, 1 H, CHO), 2.42 (td, *J*₁ = 7.0 Hz, *J*₂ = 1.9 Hz, 2 H, CH₂), 1.68-1.56 (m, 2 H, CH₂), 1.37-1.20 (m, 24 H, 12×CH₂), 0.88 (t, *J =* 7.0 Hz, 3 H, CH₃); **¹³C NMR** (100 MHz, CDCl₃): δ = 202.9, 43.9, 31.9, 29.7, 29.64, 29.62, 29.61, 29.5, 29.4, 29.3, 29.1, 22.7, 22.0, 14.1.

### Example 45

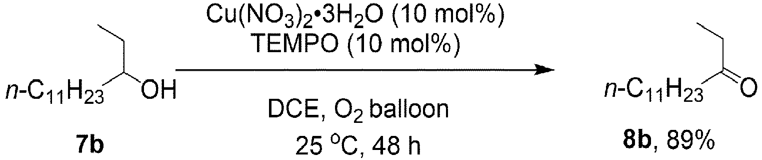

Operations were conducted by referring to Example 1. Cu(NO₃)₂•3H₂O (24.1 mg, 0.1 mmol), TEMPO (16.0 mg, 0.1 mmol), **7b** (213.3 mg, 1.0 mmol), DCE(4mL), reacted 48 hours to afford **8b** (188.3 mg, 89%) (eluent: petroleum ether/ethyl ether = 60/1): white solid. Melting point: 32.6-33.5 °C (petroleum ether/ethyl acetate recrystallization); **¹H NMR** (400 MHz, CDCl₃): δ = 2.46-2.34 (m, 4 H, 2×CH₂), 1.62-1.52 (m, 2 H, CH₂), 1.34-1.18 (m, 16 H, 8×CH₂), 1.05 (t, *J =* 7.2 Hz, 3 H, CH₃), 0.88 (t, *J =* 6.8 Hz, 3 H, CH₃); **¹³C NMR** (100 MHz, CDCl₃): δ = 211.8, 42.3, 35.7, 31.8, 29.5, 29.4, 29.35, 29.25, 29.2, 23.9, 22.6, 14.0, 7.7; **MS** (70 eV, EI) *m*/*z* (%): 213 (M⁺+1, 1.00), 212 (M⁺, 2.03), 72 (100); **IR** (neat): v = 2960, 2916, 2872, 2849, 1709, 1702, 1471, 1463, 1455, 1374, 1231, 1131, 1114 cm⁻¹.

### Example 46

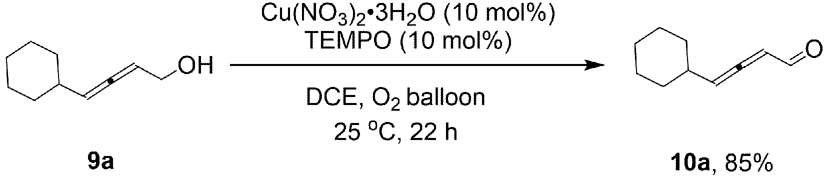

Operations were conducted by referring to Example 1. Cu(NO₃)₂•3H₂O (24.4 mg, 0.1 mmol), TEMPO (16.0 mg, 0.1 mmol), **9a** (152.0 mg, 1.0 mmol), DCE(4mL), reacted 22 hours to afford **10a** (128.2 mg, 85%) (eluent: petroleum ether/ethyl ether = 60/1): oily liquid; **¹H NMR** (400 MHz, CDCl₃): δ = 9.47 (d, *J* = 7.2 Hz, 1 H), 5.89-5.81 (m, 1 H), 5.75 (t, *J =* 6.0 Hz, 1 H), 2.28-2.13 (m, 1 H), 1.92-1.56 (m, 5 H), 1.40-1.08 (m, 5 H); **¹³C NMR** (100 MHz, CDCl₃): δ = 218.5, 192.3, 102.0, 99.4, 36.6, 32.8, 32.7, 25.7.

### Example 47

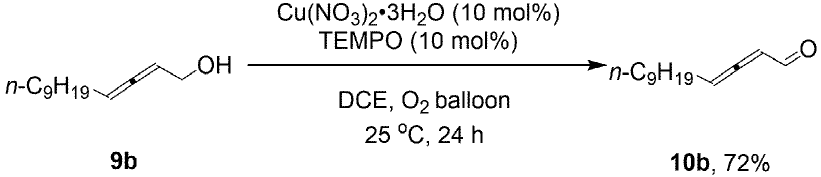

Operations were conducted by referring to Example 1. Cu(NO₃)₂•3H₂O (24.0 mg, 0.1 mmol), TEMPO (16.0 mg, 0.1 mmol), **9b** (198.1 mg, 1.0 mmol), DCE (4 mL), reacted 24 hours to afford **10b** (141.8 mg, 72%) (eluent: petroleum ether/ethyl ether = 60/1): oily liquid; **¹H NMR** (400 MHz, CDCl₃): δ = 9.49 (d, *J* = 7.2 Hz, 1 H), 5.85-5.71 (m, 2 H), 2.24-2.14 (m, 2 H), 1.54-1.42 (m, 2 H), 1.42-1.20 (m, 12 H, 6×CH₂), 0.88 (t, *J =* 6.6 Hz, 3 H); **¹³C NMR** (100 MHz, CDCl₃): δ = 219.1, 192.3, 98.6, 96.3, 31.8, 29.5, 29.25, 29.21, 28.9, 28.8, 27.4, 22.6, 14.0.

### Example 48

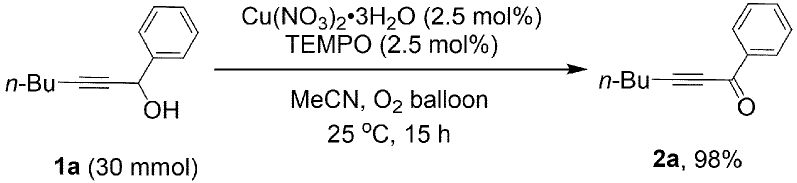

An oxygen balloon was inserted into the dry 250 mL flask, pumped O₂ for three times, Cu(NO₃)₂•3H₂O (182.3 mg, 0.75 mmol), TEMPO (120.3 mg, 0.75 mmol), **1a** (5.6549 g, 30 mmol) of MeCN solution (30 mL) were added sequentially. The flask was stirred at 25 °C for 15 h. The mixture solution was filtered through a short column of silica gel, and washed with ethyl ether, rotary evaporation to remove the solvent. The mixture solution was separated and purified by column chromatography on silica gel (eluent: petroleum ether/ethyl ether = 30/1), to afford **2a** (5.4919 g, 98%): oily liquid; **¹H NMR** (400 MHz, CDCl₃): δ = 8.14 (d, *J =* 7.2 Hz, 2 H, ArH), 7.60 (t, *J* = 7.4 Hz, 1 H, ArH), 7.48 (t, *J =* 7.6 Hz, 2 H, ArH), 2.51 (t, *J =* 7.0 Hz, 2 H, CH₂), 1.73-1.60 (m, 2 H, CH₂), 1.57-1.45 (m, 2 H, CH₂), 0.97 (t, *J =* 7.4 Hz, 3 H, CH₃); **¹³C NMR (100** MHz, CDCl₃): δ = 178.1, 136.8, 133.8, 129.4, 128.4, 96.8, 79.6, 29.7, 22.0, 18.8, 13.4.

### Example 49

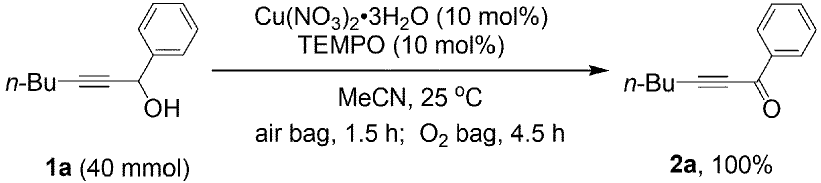

Cu(NO₃)₂•3H₂O (968.2 mg, 4.0 mmol), TEMPO (636.7 mg, 4.0 mmol), **1a** (7.5332 g, 40.0 mmol) of MeCN solution (80 mL) were added sequentially into a dry 250 mL three-necked flask. A 42 L airbag was connected to the three-necked flask, and the three-necked flask was stirred at 25 °C, after 1.5 hours, a 2 L O₂ bag was connected to the three-necked flask to supplement oxygen. The three-necked flask was stirred at 25 °C for 4.5 h. The mixture solution was filtered through a short column of silica gel, and then washed with ethyl ether, rotary evaporation to remove solvent. The mixture solution was separated and purified by column chromatography on silica gel (eluent: petroleum ether/ethyl ether = 30/1), to afford **2a** (7.4587 g, 100%): oily liquid; **¹H NMR** (400 MHz, CDCl₃): δ = 8.14 (d, *J =* 8.0 Hz, 2 H, ArH), 7.60 (t, *J =* 7.4 Hz, 1 H, ArH), 7.48 (t, *J =* 7.6 Hz, 2 H, ArH), 2.51 (t, *J =* 7.2 Hz, 2 H, CH₂), 1.74-1.62 (m, 2 H, CH₂), 1.58-1.46 (m, 2 H, CH₂), 0.97 (t, *J =* 7.4 Hz, 3 H, CH₃); **¹³C NMR** (100 MHz, CDCl₃): δ = 178.1, 136.8, 133.8, 129.4, 128.4, 96.7, 79.6, 29.7, 21.9, 18.8, 13.4.

### Example 50

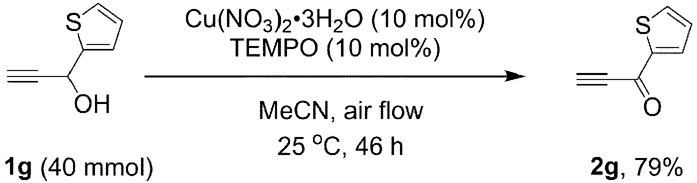

Cu(NO₃)₂•3H₂O (967.8 mg, 4.0 mmol), TEMPO (639.4 mg, 4.0 mmol), **1g** (5.5340 g, 40.0 mmol) of MeCN solution (120 mL) were added sequentially into a dry 250 mL three-necked flask. The three-necked flask was connected with the air cylinder and the air flow released slowly (high purity air, 30 mL/min), The three-necked flask was stirred at 25 °C for 46 h. The mixture solution was filtered through a short column of silica gel, and then washed with ethyl ether, rotary evaporation to remove solvent. The mixture solution was separated and purified by column chromatography on silica gel (eluent: petroleum ether/ethyl ether = 30/1), to afford **2g** (4.3224 g, 79%): white solid; ¹**H NMR** (400 MHz, CDCl₃): δ = 7.98 (d, *J*₁ *=* 3.6 Hz, 1 H, ArH), 7.75 (d, *J =* 4.8 Hz, 1 H, ArH), 7.18 (t, *J =* 4.2 Hz, 1 H, ArH), 3.36 (s, 1 H, ≡CH); ¹³**C NMR** (100 MHz, CDCl₃): δ = 169.0, 144.0, 136.1, 135.9, 128.4, 79.8, 79.4.

### Example 51

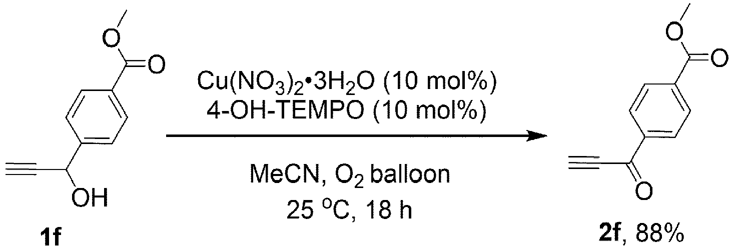

Operations were conducted by referring to Example 1. Cu(NO₃)₂•3H₂O (24.4 mg, 0.1 mmol), 4-OH-TEMPO (18.0 mg, 0.1 mmol), **1f** (190.4 mg, 1.0 mmol), MeCN (4 mL), reacted 18 hours to afford **2f** (166.3 mg, 88%) (eluent: petroleum ether/ethyl ether = 30/1): white solid. **¹H NMR** (400 MHz, CDCl₃) δ = 8.22 (d, *J =* 7.6 Hz, 2 H, ArH), 8.16 (d, *J =* 7.6 Hz, 2 H, ArH), 3.97 (s, 3 H, CH₃), 3.52 (s, 1 H, ≡CH); **¹³C NMR** (100 MHz, CDCl₃): δ = 176.6, 166.0, 139.0, 135.0, 129.8, 129.5, 81.7, 80.0, 52.5.

### Example 52

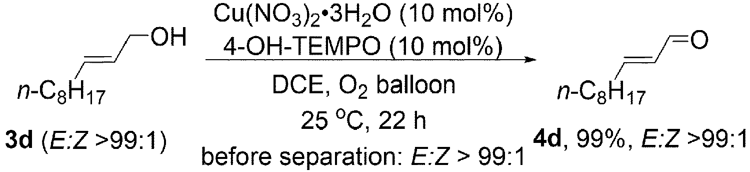

Operations were conducted by referring to Example 1. Cu(NO₃)₂•3H₂O (24.4 mg, 0.1 mmol), 4-OH-TEMPO (17.4 mg, 0.1 mmol), **3d** (177.6 mg, 1.0 mmol), DCE (4 mL), reacted 22 hours to afford **4d** (166.2 mg, 99%, E:Z >99:1) (eluent: petroleum ether/ethyl ether = 60/1) (before separation afforded a crude mixture E:Z > 99:1, as determined by ¹H NMR analysis): oily liquid; **¹H NMR** (400 MHz, CDCl₃): δ = 9.51 (d, *J =* 7.6 Hz, 1 H, CHO), 6.85 (dt, *J*₁ = 15.6 Hz, *J =* 7.0 Hz, 1 H, CH=), 6.18-6.07 (dd, *J*₁ = 15.6 Hz, *J* = 8.0 Hz, 1 H, CH=), 2.34 (dd, *J*₁ = 14.4 Hz, *J* = 7.2 Hz, 2 H, CH₂), 1.56-1.44 (m, 2 H, CH₂), 1.40-1.20 (m, 10 H, 5×CH₂), 0.89 (t, *J=* 6.6 Hz, 3 H, CH₃); **¹³C NMR** (100 MHz, CDCl₃): δ = 194.0, 158.9, 132.9, 32.6, 31.7, 29.2, 29.05, 29.03, 27.7, 22.5, 14.0.

### Example 53

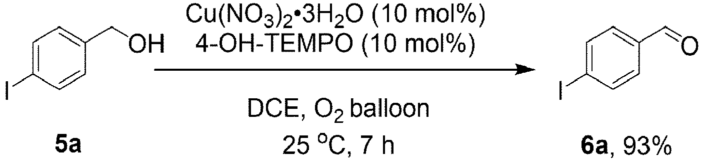

Operations were conducted by referring to Example 1. Cu(NO₃)₂•3H₂O (24.4 mg, 0.1 mmol), 4-OH-TEMPO (17.9 mg, 0.1 mmol), **5a** (241.0 mg, 1.0 mmol), DCE(4 mL), reacted 7 hours to afford **6a** (215.6 mg, 93%) (eluent: petroleum ether/ethyl ether = 30/1): white solid. **¹H NMR** (400 MHz, CDCl₃): δ = 9.96 (s, 1 H, CHO), 7.92 (d, *J* = 8.0 Hz, 2 H, ArH), 7.59 (d, *J* = 8.0 Hz, 2 H, ArH); **¹³C NMR** (100 MHz, CDCl₃): δ= 191.4, 138.4, 135.5, 130.8, 102.8.

### Example 54

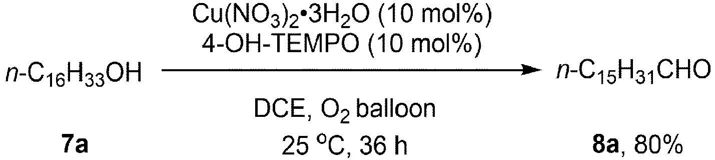

Operations were conducted by referring to Example 1. Cu(NO₃)₂•3H₂O (24.3 mg, 0.1 mmol), 4-OH-TEMPO (17.6 mg, 0.1 mmol), **7a** (247.1 mg, 1.0 mmol), DCE (4 mL), reacted 36 hours to afford **8a** (192.7 mg, 80%) (eluent: petroleum ether/ethyl ether = 60/1): white solid. **¹H NMR** (400 MHz, CDCl₃): δ = 9.76 (s, 1 H, CHO), 2.42 (t, *J* = 7.2 Hz, 2 H, CH₂), 1.63 (t, *J* = 6.8 Hz, 2 H, CH₂), 1.37-1.18 (m, 24 H, 12×CH₂), 0.88 (t, *J* = 6.6 Hz, 3 H, CH₃); **¹³C NMR** (100 MHz, CDCl₃): δ = 202.8, 43.9, 31.9, 29.6, 29.65, 29.63, 29.55, 29.4, 29.3, 29.1, 22.7, 22.0, 14.1.

### Example 55

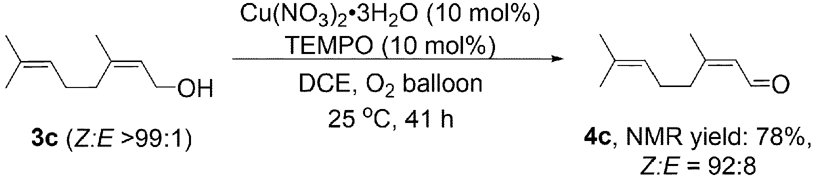

Operations were conducted by referring to Example 1. Cu(NO₃)₂•3H₂O (24.4 mg, 0.1 mmol), TEMPO (16.0 mg, 0.1 mmol), **3c** (158.5 mg, 1.0 mmol), DCE (10 mL), reacted 41 hours to afford a crude mixture of **4c,** (78% NMR yield, Z:E = 92:8, as determined by ¹H NMR analysis).

### Example 56

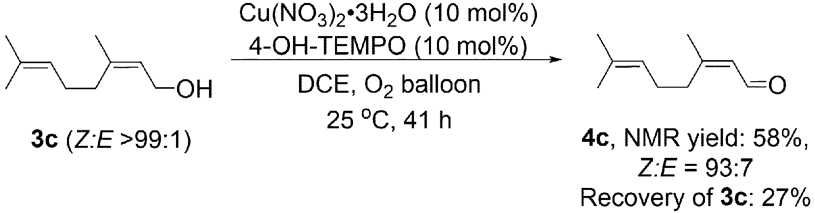

Operations were conducted by referring to Example 1. Cu(NO₃)₂•3H₂O (24.4 mg, 0.1 mmol), 4-OH-TEMPO (17.4 mg, 0.1 mmol), 3c (159.1 mg, 1.0 mmol), DCE (10 mL), reacted 41 hours to afford a crude mixture of **4c**, (58% NMR yield, Z:E = 93:7, raw material remaining 27%, as determined by ¹H NMR analysis).

### Example 57

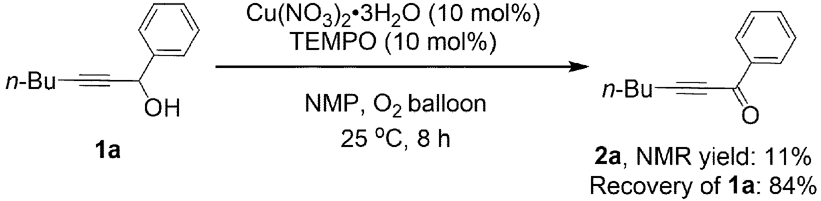

Operations were conducted by referring to Example 1. Cu(NO₃)₂•3H₂O (9.9 mg, 0.04 mmol), TEMPO (6.6 mg, 0.04 mmol), **1a** (75.4 mg, 0.4 mmol), NMP (1.6 mL), reacted 8 hours to afford **2a,** (11% NMR yield, raw material remaining 84%, as determined by ¹H NMR analysis).

### Example 58

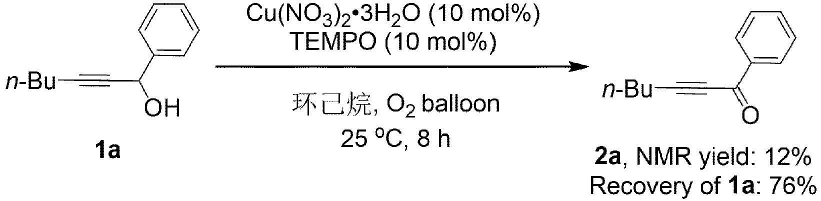

Operations were conducted by referring to Example 1. Cu(NO₃)₂•3H₂O (9.8 mg, 0.04 mmol), TEMPO (6.5 mg, 0.04 mmol), **1a** (75.2 mg, 0.4 mmol), cyclohexane (1.6 mL), reacted 8 hours to afford **2a,** (12% NMR yield, raw material remaining 76%, as determined by ¹H NMR analysis).

### Example 59

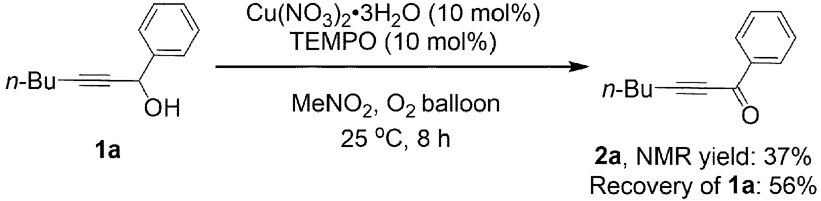

Operations were conducted by referring to Example 1. Cu(NO₃)₂·3H₂O (9.8 mg, 0.04 mmol), TEMPO (6.6 mg, 0.04 mmol), **1a** (74.9 mg, 0.4 mmol), MeNO₂ (1.6 mL), reacted 8 hours to afford **2a,** (37% NMR yield, raw material remaining 56%, as determined by ¹H NMR analysis).

### Example 60

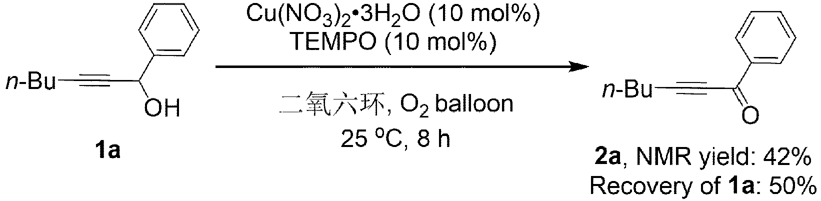

Operations were conducted by referring to Example 1. Cu(NO₃)₂•3H₂O (9.9 mg, 0.04 mmol), TEMPO (6.4 mg, 0.04 mmol), **1a** (75.2 mg, 0.4 mmol), dioxane (1.6 mL), reacted 8 hours to afford **2a,** (42% NMR yield, raw material remaining 50%, as determined by ¹H NMR analysis).

### Example 61

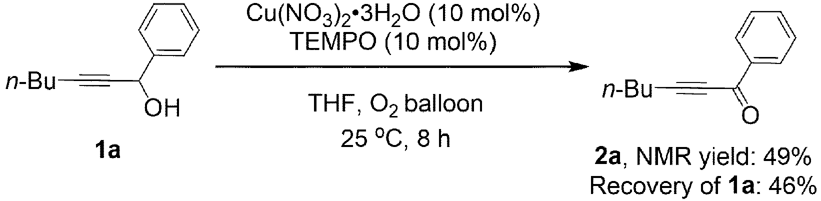

Operations were conducted by referring to Example 1. Cu(NO₃)₂•3H₂O (9.7 mg, 0.04 mmol), TEMPO (6.4 mg, 0.04 mmol), **1a** (75.7 mg, 0.4 mmol), THF (1.6 mL), reacted 8 hours to afford **2a,** (49% NMR yield, raw material remaining 46%, as determined by ¹H NMR analysis).

### Example 62

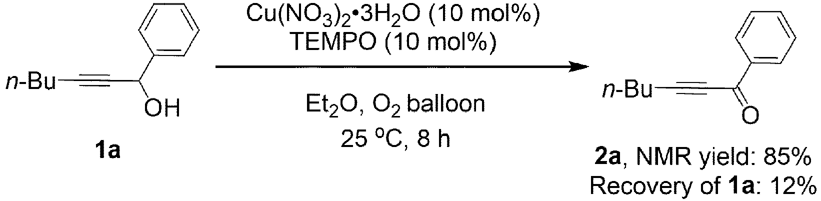

Operations were conducted by referring to Example 1. Cu(NO₃)₂•3H₂O (9.8 mg, 0.04 mmol), TEMPO (6.4 mg, 0.04 mmol), **1a** (75.2 mg, 0.4 mmol), Et₂O (1.6 mL), reacted 8 hours to afford **2a,** (85% NMR yield, raw material remaining 12%, as determined by ¹H NMR analysis).

### Example 63

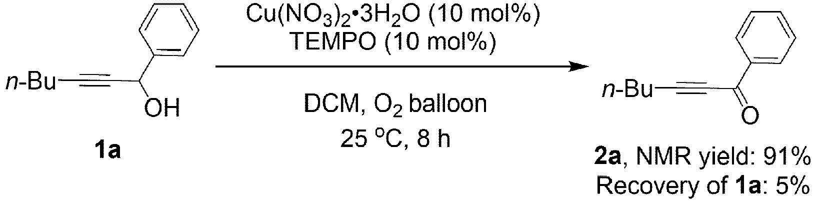

Operations were conducted by referring to Example 1. Cu(NO₃)₂•3H₂O (9.8 mg, 0.04 mmol), TEMPO (6.4 mg, 0.04 mmol), **1a** (75.0 mg, 0.4 mmol), DCM (1.6 mL), reacted 8 hours to afford **2a,** (91% NMR yield, raw material remaining 5%, as determined by ¹H NMR analysis).

### Example 64

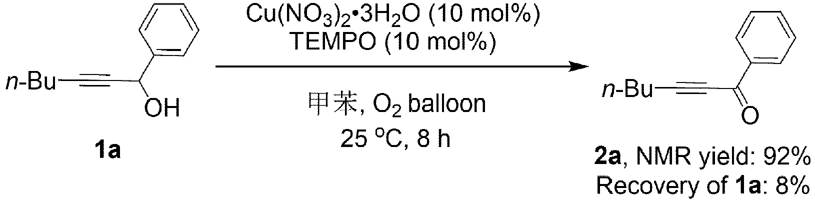

Operations were conducted by referring to Example 1. Cu(NO₃)₂•3H₂O (9.9 mg, 0.04 mmol), TEMPO (6.4 mg, 0.04 mmol), **1a** (75.0 mg, 0.4 mmol), toluene (1.6 mL), reacted 8 hours to afford **2a,** (92% NMR yield, raw material remaining 8%, as determined by ¹H NMR analysis).

### Example 65

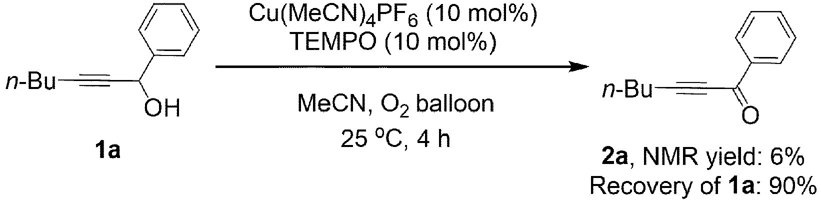

Operations were conducted by referring to Example 1. Cu(MeCN)₄PF₆ (14.9 mg, 0.04 mmol), TEMPO (6.4 mg, 0.04 mmol), **1a** (75.3 mg, 0.4 mmol), MeCN (1.6 mL), reacted 4 hours to afford **2a,** (6% NMR yield, raw material remaining 90%, as determined by ¹H NMR analysis).

### Example 66

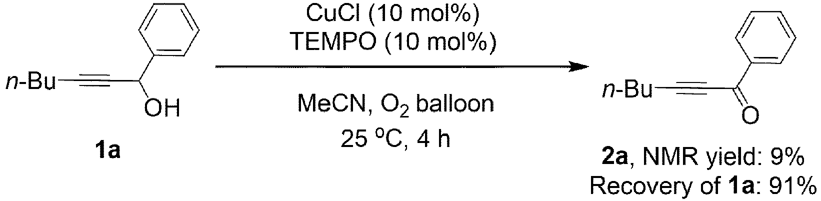

Operations were conducted by referring to Example 1. CuCl (4.0 mg, 0.04 mmol), TEMPO (6.4 mg, 0.04 mmol), **1a** (75.3 mg, 0.4 mmol), MeCN (1.6 mL), reacted 4 hours to afford **2a,** (9% NMR yield, raw material remaining 91%, as determined by ¹H NMR analysis).

### Example 67

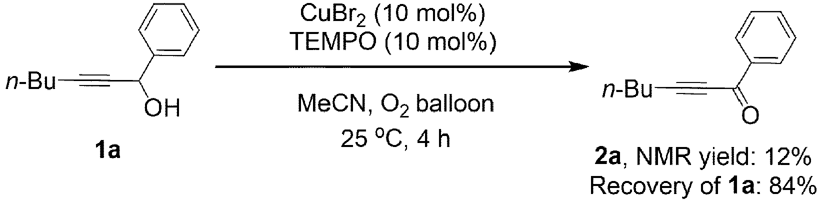

Operations were conducted by referring to Example 1. CuBr₂ (8.9 mg, 0.04 mmol), TEMPO (6.4 mg, 0.04 mmol), **1a** (75.4 mg, 0.4 mmol), MeCN (1.6 mL), reacted 4 hours to afford **2a,** (12% NMR yield, raw material remaining 84%, as determined by ¹H NMR analysis).

### Example 68

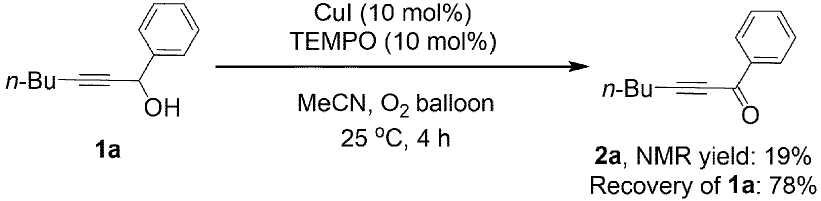

Operations were conducted by referring to Example 1. CuI (7.7 mg, 0.04 mmol), TEMPO (6.6 mg, 0.04 mmol), **1a** (75.3 mg, 0.4 mmol), MeCN (1.6 mL), reacted 4 hours to afford **2a,** (19% NMR yield, raw material remaining 78%, as determined by ¹H NMR analysis).

### Example 69

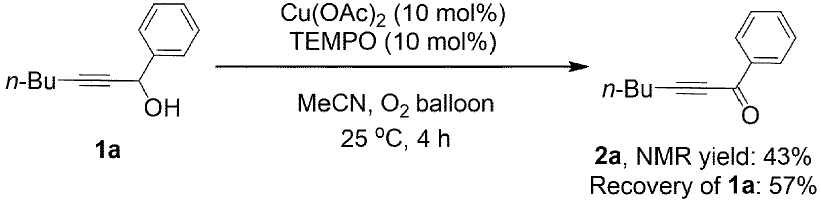

Operations were conducted by referring to Example 1. Cu(OAc)₂ (7.4 mg, 0.04 mmol), TEMPO (6.4 mg, 0.04 mmol), **1a** (75.3 mg, 0.4 mmol), MeCN (1.6 mL), reacted 4 hours to afford **2a,** (43% NMR yield, raw material remaining 57%, as determined by ¹H NMR analysis).

### Example 70

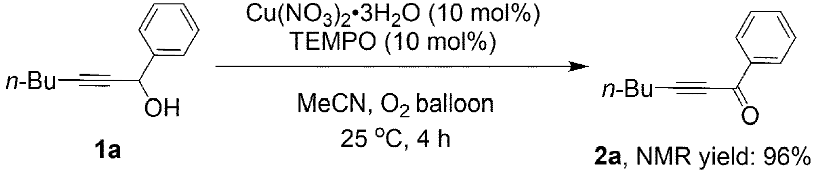

Operations were conducted by referring to Example 1. Cu(NO₃)₂•3H₂O (9.8 mg, 0.04 mmol), TEMPO (6.4 mg, 0.04 mmol), **1a** (75.4 mg, 0.4 mmol), MeCN (1.6 mL), reacted 4 hours to afford **2a,** (96% NMR yield as determined by ¹H NMR analysis).

### Example 71

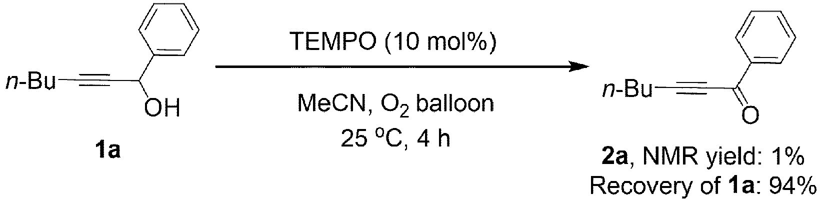

Operations were conducted by referring to Example 1. TEMPO (6.3 mg, 0.04 mmol), **1a** (75.4 mg, 0.4 mmol), MeCN (1.6 mL), reacted 4 hours to afford **2a,** (1% NMR yield, raw material remaining 94%, as determined by ¹H NMR analysis).

### Example 72

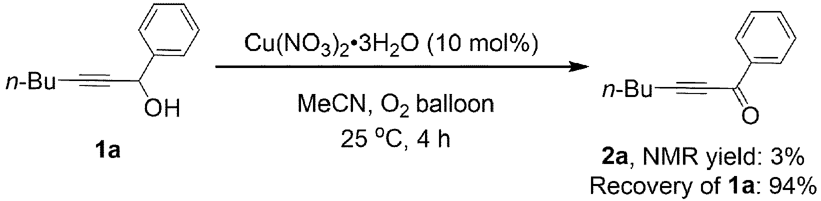

Operations were conducted by referring to Example 1. Cu(NO₃)₂•3H₂O (9.8 mg, 0.04 mmol), **1a** (75.4 mg, 0.4 mmol), MeCN (1.6 mL), reacted 4 hours to afford **2a,** (3% NMR yield, raw material remaining 94%, as determined by ¹H NMR analysis).

The present invention is not limited to the specific embodiments disclosed and described above. Without departing from the spirit and scope of the inventive concept, some modifications and changes to the present invention should also fall within the protection scope of the claims of the present invention, and the appended claims shall be the protection scope. In addition, although some specific terms are used in this specification, these terms are only for the convenience of description and do not constitute any limitation to the present invention.

## Claims

1. A copper-catalyzed method for preparing aldehydes or ketones by oxidizing alcohols with oxygen as an oxidant, wherein, at 0-100°C, in an organic solvent, using the alcohol shown in formula (a) as raw material, oxygen or oxygen in the air is used as oxidant, copper salts and nitroxide radicals are used as catalysts, reacting 4-48 hours to oxidize the alcohol to produce aldehyde or ketone compounds shown in formula (b), the reaction process has the following reaction equation (1): 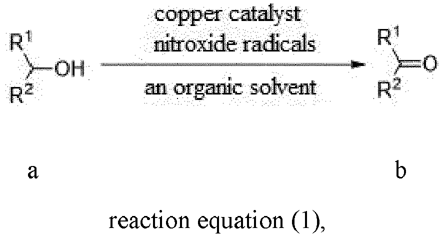 wherein, R¹ and R² is a hydrogen, an alkyl, an alkyl with functional groups, a cycloalkyl, a phenyl, an aryl, a heterocyclic group, an ethynyl, an alkynyl with functional groups, a vinyl, an alkenyl with functional groups, an allenyl, an allenyl with functional groups; the said aryl is phenyl, naphthyl, thiophene, furan, pyrrole with electron-donating or electron-withdrawing substituents at the ortho, meta, and para positions; the said heterocyclic group is thienyl, furyl or pyridyl, or thiophene, furan or pyridine with electron-donating or electron-withdrawing substituents.

2. The method of claim 1, wherein, R¹ and R² is a C1-C20 alkyl, a C1-C20 alkyl with functional groups, a C3-C8 cycloalkyl, a phenyl, an aryl, a heterocyclic group, an ethynyl, an alkynyl with functional groups, a vinyl, an alkenyl with functional groups, an allenyl, an allenyl with functional groups; the said heterocyclic group is thienyl, furyl or pyridyl, or thiophene, furan or pyridine with electron-donating or electron-withdrawing substituents;
wherein, the C1-C20 alkyl with functional groups, said the functional group is selected from carbon-carbon double bond, carbon-carbon triple bond, ester group, acyl group, acyloxy group, amide group, halogen, carboxyl group, cyano group, phenyl, aryl, thienyl, furyl; the alkynyl with functional groups, the alkenyl with functional groups, and the allenyl with functional groups, said the functional group is selected from C1-C20 alkyl, C3-C6 cycloalkyl, carbon-carbon double bond, carbon-carbon triple bond, ester group, acyl group, acyloxy group, amide group, halogen, carboxyl group, cyano group, phenyl, aryl, thienyl, furyl, silicon group;
wherein, the said aryl is phenyl, thiophene, furan, pyrrole with substituents at the ortho, meta, and para positions; the said substituent is selected from C1-C5 alkyl, ester group, hydroxyl group, acyl group, acyloxy group, nitro group, halogen, carboxyl group, cyano group, methoxyl group.

3. The method of claim 2, wherein, R1 and R2 is a C1-C20 alkyl, a C1-C20 alkyl with functional groups, a C3-C8 cycloalkyl, a phenyl, an aryl, a heterocyclic group, an ethynyl, an alkynyl with functional groups, a vinyl, an alkenyl with functional groups, an allenyl, an allenyl with functional groups; the said heterocyclic group is thienyl, furyl or pyridyl, or thiophene, furan or pyridine with electron-donating or electron-withdrawing substituents;
wherein, the C1-C20 alkyl with functional groups, the said functional group is selected from carbon-carbon double bond, carbon-carbon triple bond, methoxycarbonyl, ethoxycarbonyl, formyl, acetyl, benzoyl, formyloxy, acetoxy, benzoyloxy, acetamide, benzamide, halogen, carboxyl group, cyano group, phenyl, aryl, thienyl, furyl; the alkynyl with functional groups, the alkenyl with functional groups, and the allenyl with functional groups, said the functional group is selected from C1-C20 alkyl, C3-C6 cycloalkyl, carbon-carbon double bond, carbon-carbon triple bond, methoxycarbonyl, ethoxycarbonyl, formyl, acetyl, benzoyl, formyloxy, acetoxy, benzoyloxy, acetamide, benzamide, halogen, carboxyl group, cyano group, phenyl, aryl, thienyl, furyl, silicon group;
wherein, the said aryl is phenyl with substituents at the ortho, meta, and para positions; the said substituent is selected from C1-C5 alkyl, methoxycarbonyl, ethoxycarbonyl, hydroxyl group, formyl, acetyl, benzoyl, formyloxy, acetoxy, benzoyloxy, nitro group, halogen, carboxyl group, cyano group, methoxyl group.

4. The method of claim 1, wherein, said the method comprises the following steps:
1) inserting an oxygen balloon into the dry reaction tube, pumping air three times, and adding a copper catalyst, a nitroxide radical, an alcohol organic solvent solution in sequence, or using air to supplement oxygen, or airflow, putting the reaction tube in the 25 °C oil bath and stirring for 4-48 hours; wherein, the organic solvent is based on the amount of alcohol shown in formula (a), and the dosage of the organic solvent is 1.0-10.0 mL/mmol;
2) after the completion of the reaction in step (1), raising the reaction tube from the oil bath, filtering the mixture with silica gel short column, washing with a certain amount of diethyl ether, concentrating, and subjecting to the flash column chromatography, so as to obtain the aldehyde or ketone compounds; said the diethyl ether is based on the amount of alcohol shown in formula (a), and the dosage of the diethyl ether is 3.75-75 mL/mmol.

5. The method of claim 1, wherein the organic solvent is any one or more of benzene, toluene, dichloromethane, 1,2-dichloroethane, 1,1-dichloroethane, 1,2-dichloropropane, 1,3-dichloropropane, nitromethane, diethyl ether, ethylene glycol dimethyl ether, tetrahydrofuran or acetonitrile.

6. The method of claim 1, wherein the organic solvent is based on the amount of alcohol shown in formula (a), and the dosage of the organic solvent is 1.0-10.0 mL/mmol.

7. The method of claim 1, wherein the copper salts are any one or more of tetrakiscopper hexa-fluorophosph, cuprous chloride, copper bromide, cuprous iodide, copper acetate or copper nitrate trihydrate.

8. The method of claim 1, wherein the copper salt is based on the amount of alcohol shown in formula (a), and the dosage of the copper salt is 0.025-0.1 mmol/mmol.

9. The method of claim 1, wherein the nitroxide radicals are any one or more of 2,2,6,6-tetramethylpiperidine oxide, 4-hydroxy-2,2,6,6-tetramethylpiperidine oxide, 4-methoxy-2,2,6,6-tetramethylpiperidine oxide, 4-acetylamino -2,2,6,6-tetramethylpiperidine oxide, 4-oxy-2,2,6,6-tetramethylpiperidine oxide, 4-amino-2,2,6,6-tetramethylpiperidine oxide, N-hydroxymaleimide, 9-azabicyclo [3.3.1] nonane nitroxide radical, 2-azaadamantane nitroxide radical.

10. The method of claim 1, wherein the nitroxide radical is based on the amount of alcohol shown in formula (a), and the dosage of the nitroxide radical is 0.025-0.1 mmol/mmol.
